# EUROPEAN PATENT APPLICATION

(11) **EP 4 852 004 A1**
(43) Date of publication of application: **09.12.2026**
(21) Application number: 24922116.9
(22) Date of filing: 30.10.2024
(51) Int. Cl.: G16C 20/10, G06N 3/045

(54) **STRUCTURED PROCESSING DEVICE AND STRUCTURED PROCESSING METHOD FOR ASSISTING IN GENERATION OF STRUCTURED DATA REPRESENTING PROCESS**

(30) Priority: 30.01.2024 JP 2024011924
(71) Applicant: Hitachi, Ltd., Tokyo 100-8280 (JP)
(72) Inventor: AIKOH, Kazuhide, Tokyo 100-8280 (JP); MATSUMOTO, Shigenori, Tokyo 100-8280 (JP)
(74) Representative: Mewburn Ellis LLP
(86) International application number: PCT/JP2024/038698
(87) International publication number: WO 2025/164006

(57) **Abstract**

A structuring processing apparatus outputs structured data of a process in which a prediction target is predicted based on a process feature amount that is a feature amount based on a coarse granularity such as a process pattern of a treatment applied to the process, rather than a fine granularity such as elements in the process having a plurality of elements including a plurality of procedures.

## Description

### Technical Field

The present invention generally relates to a technology for supporting generation of structured data.

### Background Art

In recent years, in various fields, there has been a growing need to support generation of structured data representing a process including a plurality of procedures using artificial intelligence (AI) or another method. For example, AI for recommending a device operating process and recommending a process against a failure of a device has been put into practical use in the industrial field, AI for offering assistance in taking diagnosis, treatment, and medication actions has been put into practical use in the medical field, and AI for recommending a new material synthesis process has been put into practical use in the materials field.

In order to generate structured data for a new process from data accumulated in the past, an enormous amount of time, expertise, and trial and error is generally required. In this regard, the technologies described in NPL 1 and NPL 2 are known.

NPL 1 describes, as a synthetic route search method in materials informatics (MI), a method of predicting a precursor (a substance at a stage before a certain chemical substance is generated) based on route template information and structural analysis information using a graph neural network (GNN). In addition, NPL 2 describes a link estimation method based on counterfactuals, specifically, a method of estimating whether a link connection relationship changes when an attribute of a node is modified.

### Citation List

### Non Patent Literature

NPL 1: COLEY, Connor W.; GREEN, William H.; JENSEN, Klavs F. Machine learning in computer-aided synthesis planning. Accounts of chemical research, 2018, 51.5: 1281-1289.
NPL 2: ZHAO, Tong, et al. Learning from counterfactual links for link prediction. In: International Conference on Machine Learning. PMLR, 2022. p. 26911-26926.

### Summary of Invention

### Technical Problem

In order to generate structured data representing a process, prediction support processing, which is processing related to prediction processing for the process, is performed.

The technology described in NPL 1 focuses on a substance, and the technology described in NPL 2 focuses on a node. For this reason, in any of these technologies, the number of combinations may be enormous in the prediction support processing, resulting in a large computational load of the prediction support processing, and external factors of substances (nodes) (e.g., reaction conditions or synthesis purposes) may not be reflected, thereby lowering the process prediction accuracy.

The present invention has been made in view of the aforementioned problems, and an object of the present invention is to reduce a computational load of prediction support processing for a process represented by generated structured data and improve process prediction accuracy.

### Solution to Problem

A representative example of the present invention is as follows. That is, a structuring processing apparatus outputs structured data of a process in which a prediction target is predicted based on a process feature amount that is a feature amount based on a coarse granularity such as a process pattern of a treatment applied to the process, rather than a fine granularity such as elements in the process having a plurality of elements including a plurality of procedures.

### Advantageous Effects of Invention

According to the present invention, it is possible to reduce a computational load of processing for predicting a process represented by generated structured data and improve process prediction accuracy. Other problems, configurations, and effects that are not described above will be apparent from the following description of embodiments.

### Brief Description of Drawings

[FIG. 1] FIG. 1 is a diagram illustrating an example of a system according to a first embodiment.
[FIG. 2] FIG. 2 is a diagram illustrating an example of a hardware configuration of a computer.
[FIG. 3] FIG. 3 is a diagram illustrating an example of structured data.
[FIG. 4] FIG. 4 is a diagram illustrating an example of a structured data output screen.
[FIG. 5] FIG. 5 is a diagram schematically illustrating an example of structured data.
[FIG. 6] FIG. 6 is a diagram schematically illustrating an example of training processing.
[FIG. 7] FIG. 7 is a diagram schematically illustrating an example of inference processing.
[FIG. 8] FIG. 8 is a diagram illustrating an example of a reaction label.
[FIG. 9] FIG. 9 is a diagram illustrating an example of a frequent process pattern [structure] according to the first embodiment.
[FIG. 10] FIG. 10 is a diagram illustrating an example of a frequent process pattern [attribute value] according to the first embodiment.
[FIG. 11] FIG. 11 is a diagram illustrating an example of a process feature amount [structure] according to the first embodiment.
[FIG. 12] FIG. 12 is a diagram illustrating an example of a process feature amount [attribute value] according to the first embodiment.
[FIG. 13] FIG. 13 is a diagram illustrating an example of a raw material-product table.
[FIG. 14] FIG. 14 is a diagram illustrating an example of a subgraph list.
[FIG. 15] FIG. 15 is a diagram illustrating an example of a flow of causal model training processing.
[FIG. 16A] FIG. 16A is a diagram illustrating an example of a flow of frequent process pattern extraction processing.
[FIG. 16B] FIG. 16B is a diagram illustrating an example of a flow of attribute value extraction processing.
[FIG. 17] FIG. 17 is a diagram illustrating an example of a flow of process feature amount extraction processing.
[FIG. 18] FIG. 18 is a diagram illustrating an example of a flow of experimental process information registration processing.
[FIG. 19] FIG. 19 is a diagram illustrating an example of a flow of frequent process pattern matching processing according to the first embodiment.
[FIG. 20] FIG. 20 is a diagram illustrating an example of a flow of graph construction processing.
[FIG. 21] FIG. 21 is a diagram illustrating an example of a flow of process feature amount inference processing.
[FIG. 22] FIG. 22 is a diagram illustrating an example of a flow of edge prediction processing.
[FIG. 23] FIG. 23 is a diagram schematically illustrating an example of a part of training processing.
[FIG. 24A] FIG. 24A is a diagram illustrating an example of experimental process data.
[FIG. 24B] FIG. 24B is a diagram illustrating an example of a frequent process pattern [structure].
[FIG. 24C] FIG. 24C is a diagram illustrating an example of a frequent process pattern [attribute value].
[FIG. 24D] FIG. 24D is a diagram illustrating an example of a process feature amount [attribute value].
[FIG. 25] FIG. 25 is a diagram schematically illustrating an example of a part of inference processing.
[FIG. 26A] FIG. 26A is a diagram illustrating an example of a raw material-product table.
[FIG. 26B] FIG. 26B is a diagram illustrating an example of a first subgraph and a second subgraph.
[FIG. 26C] FIG. 26C is a diagram illustrating an example of a process feature amount [attribute value].
[FIG. 27] FIG. 27 is a diagram illustrating an example of measurement process data according to a second embodiment.
[FIG. 28] FIG. 28 is a diagram illustrating an example of a frequent process pattern [structure] according to the second embodiment.
[FIG. 29] FIG. 29 is a diagram illustrating an example of a frequent process pattern [attribute value] according to the second embodiment.
[FIG. 30] FIG. 30 is a diagram illustrating an example of a process feature amount [structure].
[FIG. 31] FIG. 31 is a diagram illustrating an example of a process feature amount [attribute value].
[FIG. 32] FIG. 32 is a diagram illustrating an example of a flow of frequent process pattern matching processing according to the second embodiment.
[FIG. 33A] FIG. 33A is a diagram schematically illustrating an example of matching (matching between a process pattern and process data) according to the first embodiment.
[FIG. 33B] FIG. 33B is a diagram schematically illustrating an example of matching (matching between a process pattern and process data) according to the second embodiment.

### Description of Embodiments

Hereinafter, some embodiments of the present invention will be described with reference to the drawings. The following description and drawings are examples for describing the present invention, and some omissions and simplifications have been made as appropriate for clarity of explanation. The present invention can be implemented in various other forms. Unless otherwise specified, each component may be singular or plural.

In the following description, the same or similar configurations are denoted by the same reference numerals, and redundant description may be omitted. In the following description, the letter "S" prefixed to a reference numeral denotes a processing step. In addition, in the following description, various types of information may be described using expressions "table" and the like, but the various types of information may be expressed using data structures other than such expressions.

Furthermore, in the following description, an example in which information regarding a material synthesis process described in an experimental report is structured may be adopted, but the process to be structured may be applied to various fields and use cases described in the background art.

### First Embodiment

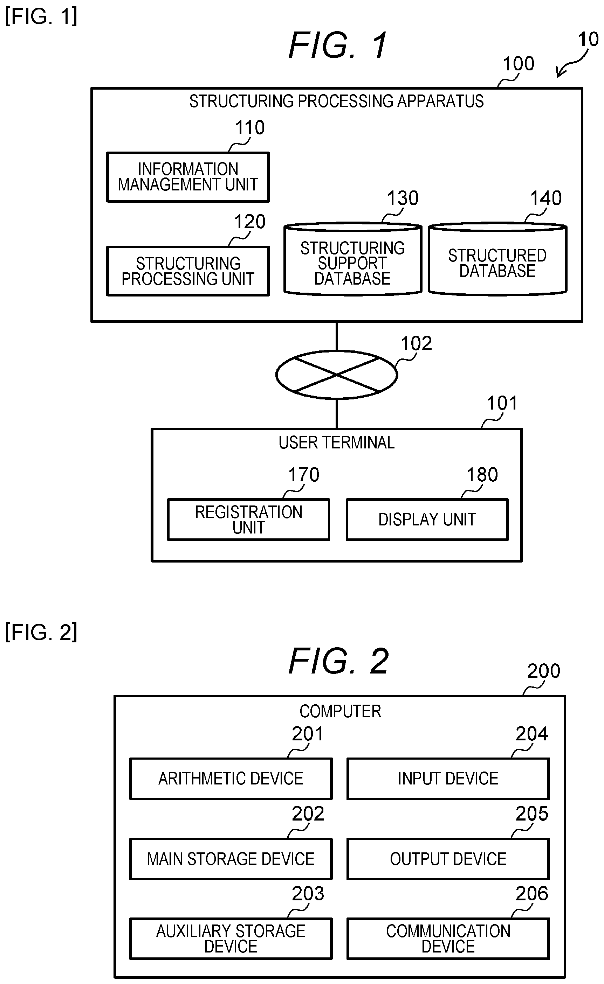

FIG. 1 is a diagram illustrating an example of a system according to the first embodiment. FIG. 2 is a diagram illustrating an example of a hardware configuration of a computer 200.

The system 10 illustrated in FIG. 1 includes a structuring processing apparatus 100 and a user terminal 101. The structuring processing apparatus 100 and the user terminal 101 are connected to each other via a communication network 102 in a bidirectionally communicable state. The communication network 102 is, for example, a local area network (LAN), a wide area network (WAN), the Internet, a public communication network, a dedicated line, or the like. Note that the number of user terminals 101 may be two or more.

The structuring processing apparatus 100 and the user terminal 101 are constituted by, for example, the computer 200 as illustrated in FIG. 2. The computer 200 includes a main storage device 202, an auxiliary storage device 203, an input device 204, an output device 205, a communication device 206, and an arithmetic device 201 connected to these devices.

The arithmetic device 201 executes a program stored in the main storage device 202. The arithmetic device 201 is, for example, a central processing unit (CPU), a micro processing unit (MPU), a graphics processing unit (GPU), a field programmable gate array (FPGA), an application specific integrated circuit (ASIC), an artificial intelligence (AI) chip, or the like. The arithmetic device 201 operates as a functional unit (module) that realizes a specific function by executing processing according to the program. In the following description, when processing is described using a functional unit as a subject, this indicates that the arithmetic device 201 executes a program for realizing the functional unit.

The main storage device 202 stores programs and data executed by the arithmetic device 201. The main storage device 202 is, for example, a read only memory (ROM), a random access memory (RAM), or a non-volatile memory such as a non-volatile RAM (NVRAM). Note that the main storage device 202 is also used as a work area.

The auxiliary storage device 203 persistently stores data. The auxiliary storage device 203 is, for example, a solid state drive (SSD), a hard disk drive, or the like. Note that the computer 200 does not need to include the auxiliary storage device 203. In this case, the programs and data may be acquired from an optical storage device such as a compact disc (CD) or a digital versatile disc (DVD), an IC card, an SD card, or the like, or may be acquired from a storage area on an externally connected storage system or a cloud system. The programs and data stored in the auxiliary storage device 203 are read by the arithmetic device 201 and loaded into the main storage device 202.

The input device 204 is an interface that receives an input from the outside. The input device 204 is, for example, a keyboard, a mouse, a touch panel, a card reader, a pen input type tablet, a voice input device, or the like.

The output device 205 is an interface that outputs various types of information such as a processing progress and a processing result. The output device 205 is, for example, a display device such as a liquid crystal monitor or a liquid crystal display (LCD), an audio output device, a printer, or the like.

Note that the computer 200 does not need to include the input device 204 and the output device 205. In this case, the computer 200 inputs and outputs information via the communication device 206.

The communication device 206 communicates with other devices. The communication device 206 is, for example, a network interface card (NIC), a wireless communication module, a USB module, or the like.

The structuring processing apparatus 100 generates structured data. Here, it is assumed that the process is, for example, an experimental process and includes a plurality of procedures. The structured data is data representing a process as a structure of a plurality of procedures, and examples of the structured data include Json format data, XML format data, RDF format data, and GraphML format data. The present invention is not limited to a data format of structured data. In the present embodiment, the structured data may be GraphML format data.

The structuring processing apparatus 100 includes an information management unit 110 and a structuring processing unit 120, and holds a structuring support database 130 and a structured data database 140. The structured data is stored in the structured data database 140. Data other than the structured data, which is referred to or generated in structuring processing performed by the structuring processing unit 120, may be stored in the structuring support database 130.

The information management unit 110 manages data stored in the structuring support database 130 and the structured data database 140. The structuring processing unit 120 executes structuring processing and stores generated structured data in the structured data database 140. Note that the information management unit 110 and the structuring processing unit 120 may be realized as one function of middleware or the like that manages an operating system, a file system, a relational database, and NoSQL such as key-value store (KVS). The structuring processing may include prediction model processing involving a prediction model for predicting a process, and processing of generating structured data representing the predicted process.

The user terminal 101 includes a registration unit 170 that displays a screen (an example of a user interface (UI)) for receiving registration, and a display unit 180 that displays a screen for presenting structured data and receiving correction thereto and the like.

Note that the functions of the structuring processing apparatus 100 may be realized using a computer system including a plurality of computers 200. In addition, all or some of the functions of the structuring processing apparatus 100 may be realized using a virtualization technology. For example, all or some of the functions of the structuring processing apparatus 100 may be realized using a cloud service such as software as a service (SaaS), platform as a service (PaaS), or infrastructure as a service (IaaS). Furthermore, the functions of the user terminal 101 may be integrated into the structuring processing apparatus 100, and the user terminal 101 may be a user interface device for input and output.

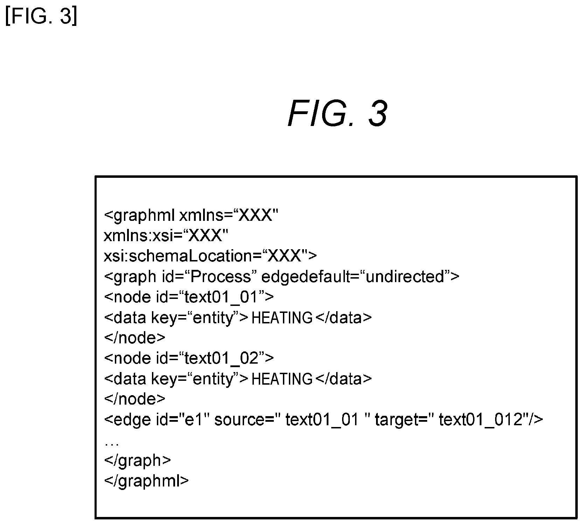

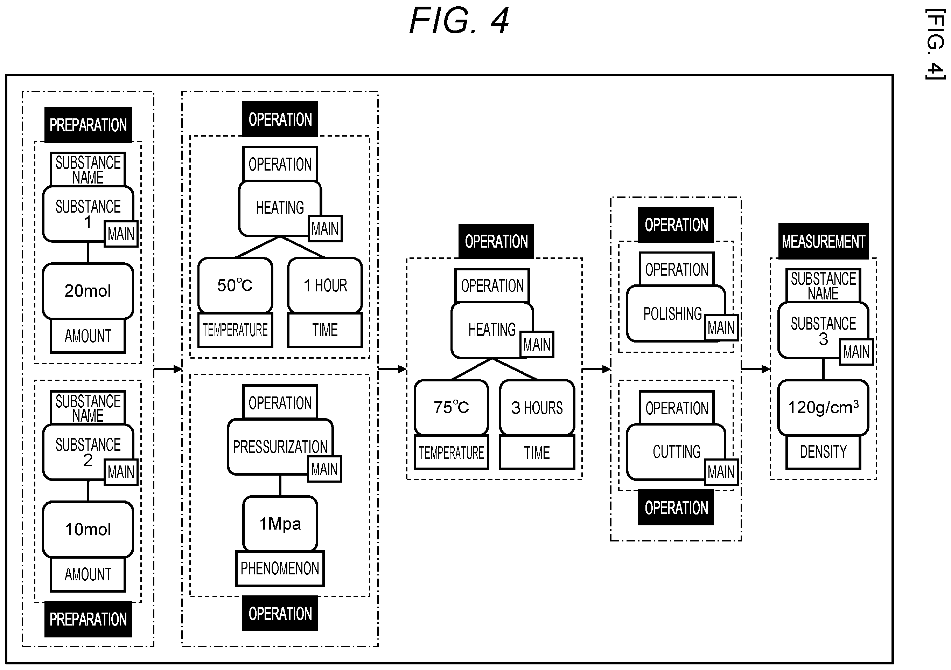

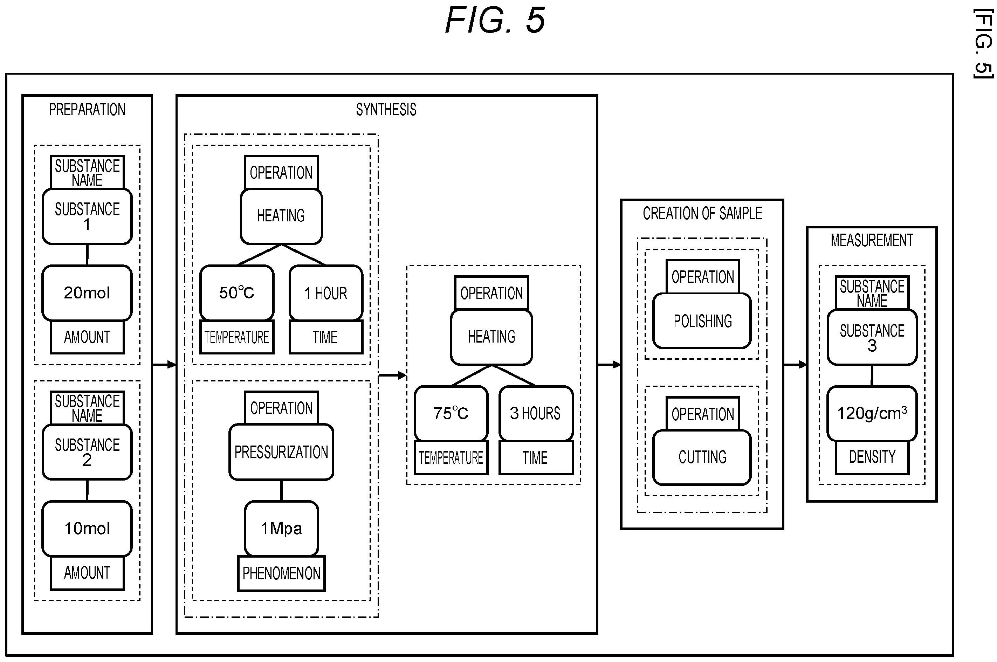

As exemplified in FIG. 3, the structured data is GraphML format data including data representing nodes in a graph and edges connecting the nodes. The display unit 180 of the user terminal 101 displays a screen displaying a graph represented by structured data, for example, a screen exemplified in FIG. 4. FIG. 5 schematically illustrates an example of a graph represented by structured data.

Through MI-related technology, it is possible to provide a recipe as data including a pair of a product (e.g., a material) and a raw material necessary for generating the product. In the present embodiment, the structuring processing unit 120 predicts a process for generating a product represented by a recipe from raw materials represented by the recipe by performing retrosynthetic analysis, and generates structured data representing the process. The retrosynthetic analysis processing is included in the structuring processing. The structuring processing performed in the present embodiment can be roughly divided into training processing and inference processing. Hereinafter, the training processing and the inference processing will be described. In the following description, structured data is graph data representing a graph including nodes and edges. The graph may be a directed graph or an undirected graph.

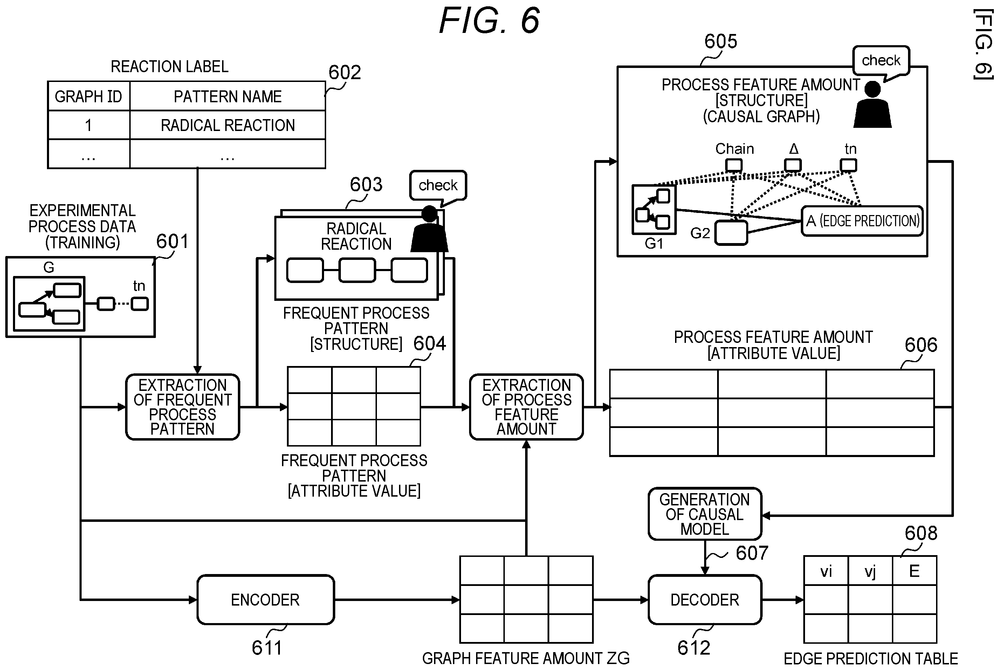

FIG. 6 is a diagram schematically illustrating an example of training processing.

The structuring processing unit 120 generates a frequent process pattern [structure] 603 and a frequent process pattern [attribute value] 604 by executing frequent process pattern extraction processing based on experimental process data 601 and reaction label 602. The experimental process data 601 is structured data representing a completed experimental process performed in the past. The reaction label 602 is data representing a correspondence relationship between an experimental process and a pattern applied to the experimental process. The frequent process pattern [structure] 603 is structured data representing a frequent process pattern. The frequent process pattern is a process portion that is common to a plurality of experimental processes. The frequent process pattern [attribute value] 604 is attribute value data of the frequent process pattern. The experimental process data 601 may be stored in the structured data database 140. The reaction label 602 may be stored in the structuring support database 130. The frequent process pattern [structure] 603 may be stored in the structured data database 140. The frequent process pattern [attribute value] 604 may be stored in the structuring support database 130. The information management unit 110 may display a screen displaying the frequent process pattern [structure] 603 through the display unit 180 of the user terminal 101, and may receive an edit of a graph represented by the frequent process pattern [structure] 603 from the user via the screen.

Next, the structuring processing unit 120 generates a process feature amount [structure] 605 and a process feature amount [attribute value] 606 by executing process feature amount extraction processing using the frequent process pattern [structure] 603, the frequent process pattern [attribute value] 604, the experimental process data 601, and a graph feature amount ZG (a graph feature amount obtained from the experimental process data 601). The process feature amount [structure] 605 is structured data representing a causal graph of the process feature amount. The process feature amount [attribute value] 606 is attribute value data of the process feature amount. Note that the information management unit 110 may display a screen displaying the process feature amount [structure] 605 through the display unit 180 of the user terminal 101, and receive an edit of the causal graph of the process feature amount from the user via the screen. The process feature amount [structure] 605 may be stored in the structured data database 140. The process feature amount [attribute value] 606 may be stored in the structuring support database 130.

Next, the structuring processing unit 120 generates a causal model 607 by performing causal model generation processing based on the generated process feature amount [structure] 605 and process feature amount [attribute value] 606. The process feature amount [attribute value] 606 may be stored in the structuring support database 130.

Finally, the structuring processing unit 120 trains an encoder 611 that extracts a graph feature amount ZG from the experimental process data 601, and a decoder 612 that performs edge prediction between subgraphs using the graph feature amount ZG and the causal model 607. A model serving as the encoder 611 and a model serving as the decoder 612 may be stored in the structuring support database 130. Structured data is input to the encoder 611, and the encoder 611 outputs a graph feature amount ZG of the input structured data (graph data). A causal model and a graph feature amount are input to the encoder 611, and the encoder 611 outputs an edge prediction table 608. The training of the encoder 611 and the decoder 612 may be performed, for example, as follows.
- The structuring processing unit 120 trains the encoder 611 and the decoder 612 by using data including the experimental process data 601 as train data and data including the edge prediction table 608 as correct answer data.
- Thereafter, the structuring processing unit 120 replaces the decoder 612 trained in the present processing with the causal model 607. Specifically, the model obtained as a result of training the decoder 612 is a model [E=f(ZG)] that predicts an edge from experimental process data, whereas the causal model is a model [E=f(ZG, process feature amount)] that predicts an edge using experimental process data and a process feature amount. By replacing the decoder 612 with the causal model 607, the same problem can be solved with more explanatory variables.

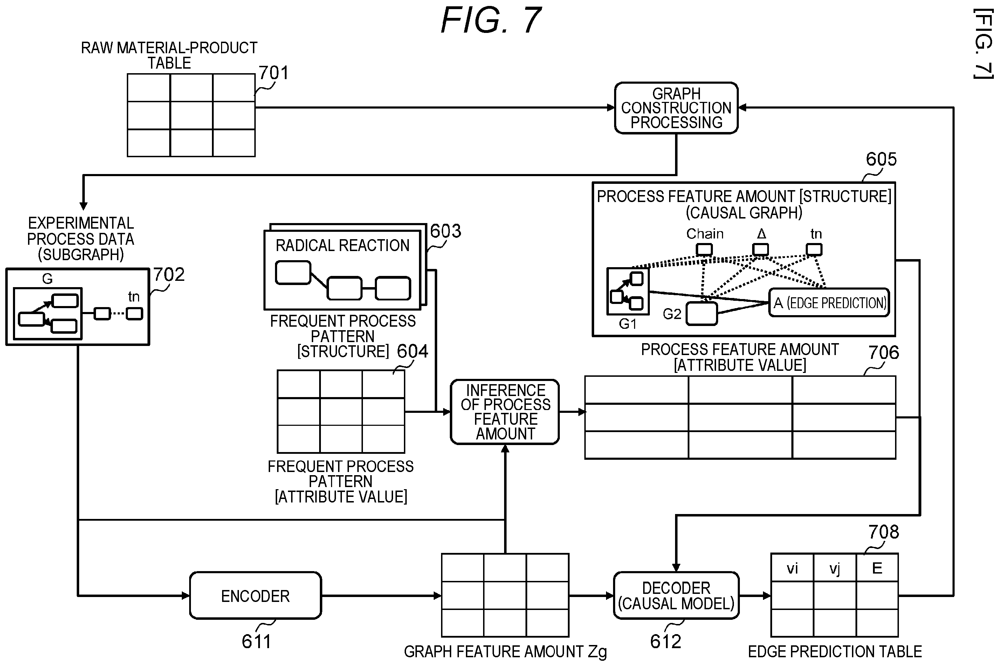

FIG. 7 is a diagram schematically illustrating an example of inference processing.

The structuring processing unit 120 inductively generates subgraphs by executing graph construction processing based on a raw material-product table 701 (a table representing a combination of a raw material and a product for identifying an experimental process). As a result, the subgraphs are expanded by connecting the subgraphs to each other using edges, thereby generating experimental process data 702 as a graph or its subgraphs. The raw material-product table 701 may be data given from the user terminal 101, or may be stored in the structuring support database 130. The experimental process data 702 as a graph (i.e., completed data) represents an experimental process for obtaining a product represented by the raw material-product table 701 from a raw material represented by the same table 701.

In this graph construction processing, the structuring processing unit 120 executes process feature amount inference processing based on a graph feature amount Zg (a graph feature amount obtained from the experimental process data 702) and the frequent process pattern [structure] 603 and the frequent process pattern [attribute value] 604 generated in the training processing, thereby generating a process feature amount [attribute value] 706, which is attribute value data of the process feature amount of the subgraph. The process feature amount [structure] 605 is a process feature amount [configuration] 605 generated in the training processing. The configuration of the process feature amount [attribute value] 706 may be the same as the configuration of the process feature amount [attribute value] 606 in the training processing. The process feature amount [attribute value] 706 may be stored in the structuring support database 130. Next, the structuring processing unit 120 generates a graph feature amount Zg of the subgraph using the encoder 611 trained in the training processing, and inputs the graph feature amount Zg and a value obtained from the process feature amount [structure] 605 and the process feature amount [attribute value] 706 to the decoder 612 trained in the training processing. As a result, an edge prediction table 708 representing a prediction result is obtained, for each pair of a node in a first subgraph and a node in a second subgraph, as to whether or not the nodes constituting the pair are connected by an edge. In the graph construction processing, the subgraphs can be expanded by applying the edge prediction table 708 to the first subgraph and the second subgraph. For example, for a pair of a certain node in the first subgraph and a certain node in the second subgraph, when the edge prediction table 708 indicates that there is an edge (that is, when it is predicted that there is an edge), the nodes are connected by the edge, so that the first subgraph and the second subgraph are connected by the edge, and as a result, the subgraphs expand. In the graph construction processing, the structuring processing unit 120 sets the expanded subgraph as a first subgraph and another subgraph as a second subgraph, performs process feature amount inference processing and causal model generation processing, and inputs a graph feature amount Zg generated by the encoder 611 for the first subgraph and a causal model 707 generated through the causal model generation processing to the decoder 612, thereby obtaining a prediction result as to whether or not a node in the first subgraph and a node in the second subgraph are connected to each other by an edge.

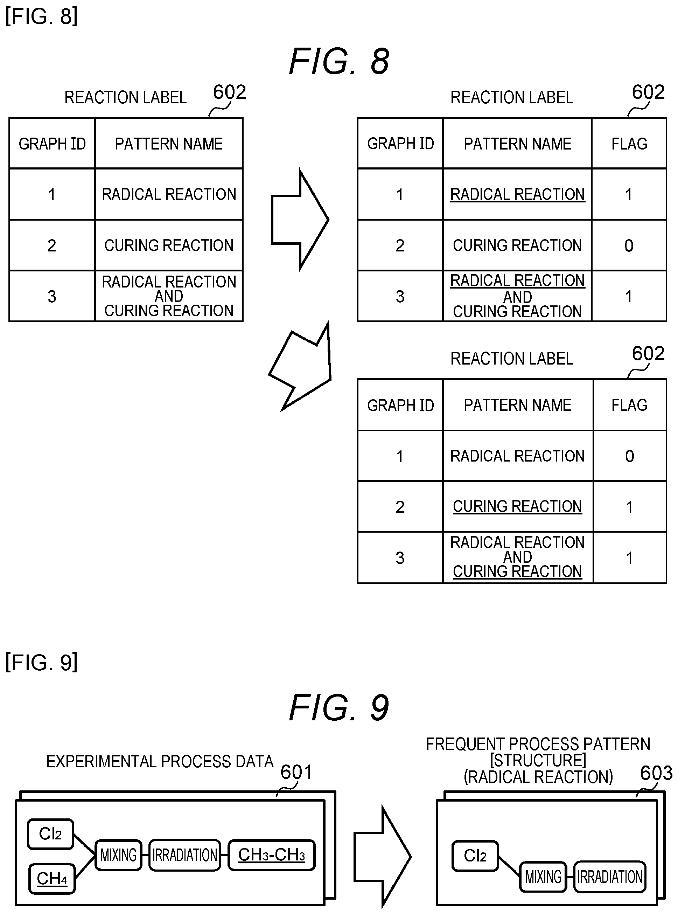

FIG. 8 is a diagram illustrating an example of the reaction label 602.

The reaction label 602 has a graph ID field and a pattern name field. In the graph ID field, a graph ID is registered as an ID of an experimental process. In the pattern name field, a name of a reaction to be applied to the experimental process is registered as a pattern name (label). For example, the first record indicates that a radical reaction is applied to an experimental process to which the graph ID "1" is assigned.

Furthermore, in the frequent process pattern extraction processing, the structuring processing unit 120 may prepare a flag (alternatively, a reaction label to which a flag field is added) for each treatment (reaction). In the flag field, a value indicating whether or not the treatment (reaction) designated by the pattern name exists in the graph is registered. For example, it is indicated that the radical reaction is applied to graphs having graph IDs 1 and 3, and the effect reaction is applied to graphs having graph IDs 2 and 3.

FIG. 9 is a diagram illustrating an example of the frequent process pattern [structure] 603.

The frequent process pattern [structure] is structured data representing a partial structure common between experimental processes. For example, in the example illustrated in the drawing, there is a common pattern in a plurality of experimental processes in which "a radical reaction occurs when Cl₂ and a certain substance are mixed and then irradiated with light", and structured data representing the common pattern (graph portion) is the frequent process pattern [structure] 603.

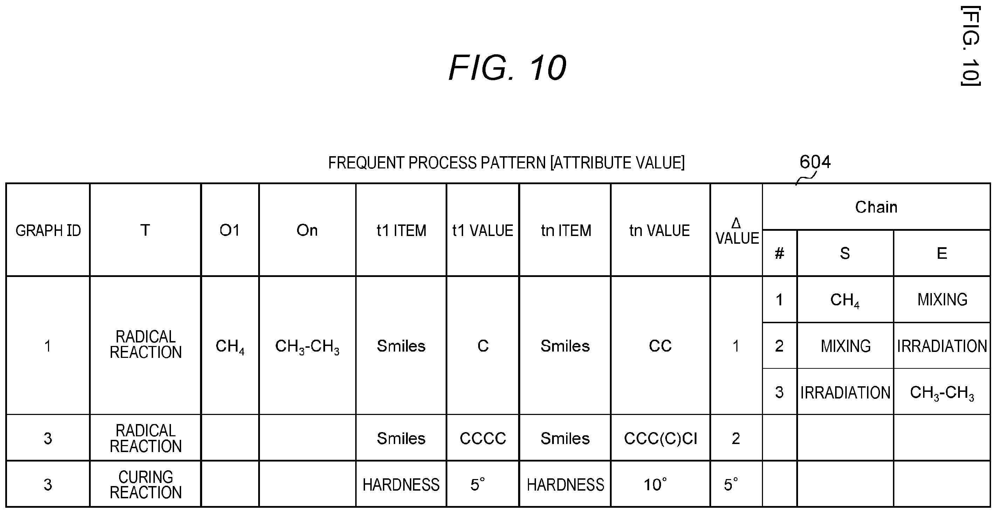

FIG. 10 is a diagram illustrating an example of the frequent process pattern [attribute value] 604.

The frequent process pattern [attribute value] 604 includes a graph ID field, a T field, an O1 field, an On field, a t1 item field, a t1 value field, a tn item field, a tn value field, a Δ value field, and a Chain field.

In the graph ID field, a graph ID is registered as an ID of an experimental process. In the T field, a name of a treatment is registered as a reaction applied to the experimental process.

In the O1 field, a name of a substance to which the reaction is to be applied is registered. In the On field, a name of a substance generated as a result of applying the reaction is registered.

In the t1 item field, a name of a physical property item of a source substance t1 to be reacted is registered. In the t1 value field, a t1 value, which is a physical property value of t1, is registered. In the tn item field, a name of a physical property item of a target substance tn generated as a result of the reaction is registered. In the tn value field, a tn value, which is a physical property value of tn, is registered. The "physical property item" may be a structure item or an attribute item. The "physical property value" may be a value (structure value or attribute value) belonging to the structure item or the attribute item.

In the Δ value field, a Δ value is registered as a difference between the physical property value as the t1 value and the physical property value as the tn value.

The Chain field includes a # field, an S field, and an E field. In the # field, an edge ID is registered as a position of an edge in the frequent process pattern. In the S field, a name of an element (e.g., a substance or an operation) corresponding to a node at one end of an edge (a start point node when the edge is directed) is registered. In the E field, a name of an element (e.g., a substance or an operation) corresponding to a node at the other end of the edge (an end point node when the edge is directed) is registered.

For example, the first record indicates the following.
- In the first experimental process, when a radical reaction is applied, CH₄(O1) is changed to CH₃-CH₃(On), and the Smiles information is converted from "C" (t1 value) to "CC" (tn value), and therefore the Δ value (e.g., the character string editing distance of Smiles) is "1".
- In the frequent process pattern [structure] 603 of the radical reaction, the edge ID of the edge connecting the node "CH₄" and the node "mixing" is "1".

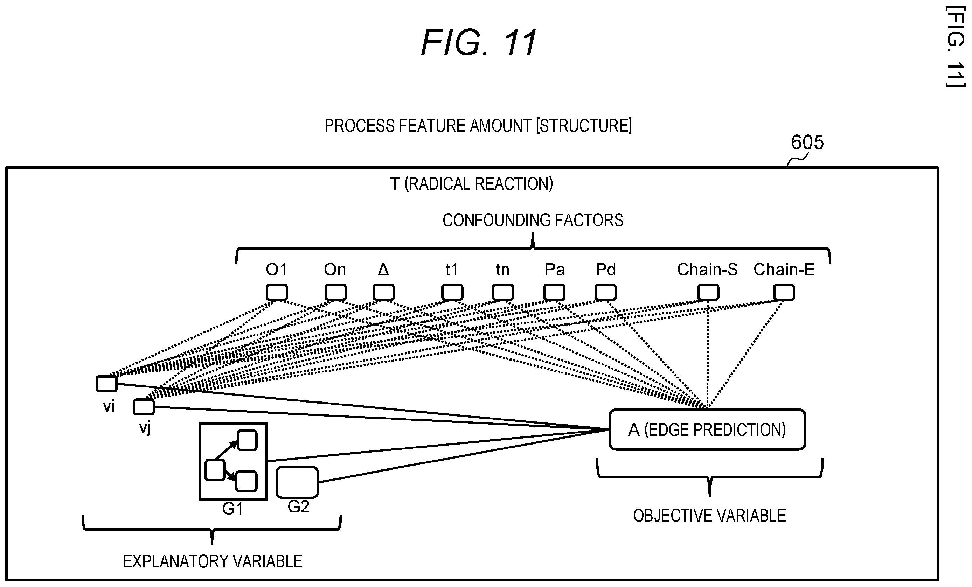

FIG. 11 is a diagram illustrating an example of the process feature amount [structure] 605.

The process feature amount [structure] 605 is structured data representing a causal graph as a relationship between a reaction (explanatory variable) extracted as a frequent process pattern and an edge existence probability (objective variable) between a first subgraph and a second subgraph. The process feature amount [structure] 605 exemplified in the drawing is as follows.
- The explanatory variable T is a radical reaction applied between a node vi in a first subgraph G1 (e.g., a subgraph of an experimental process) and a node vj in a second subgraph G2.
- The objective variable A is an edge existence probability between the nodes vi and vj to which the radical reaction is applied.
- The process feature amount is used to predict the objective variable A. The process feature amount includes a flow feature amount, a path feature amount, and a chain feature amount.
- The flow feature amount is a feature amount related to a change that occurs when a treatment T is applied to a substance O1 (e.g., a raw material) to obtain a substance On (e.g., a product) in a flow from the substance O1 to the substance On. In the present embodiment, the flow feature amount is based on the treatment (reaction) T and the Δ value as the amount of change from the O1 value to the On value.
- The path feature amount is a feature amount related to a path in which a change from t1 to tn has occurred in the flow. In the present embodiment, the path feature amount is based on Pa and Pd. Pa represents whether the subgraph G1 exists on a path to the source substance t1 (the element having the t1 value) of the treatment T, that is, whether the target substance exists in the ancestor. Pd represents whether the subgraph G2 exists on a path to the target substance tn (the element having the tn value) of the processing T, that is, whether the target substance exists in the descendant.
- The chain feature amount is based on the positions of the subgraphs G1 and G2 in the frequent process pattern. In the present embodiment, the chain feature amount is a Chain indicating positions where the subgraphs G1 and G2 exist in the frequent process pattern of the treatment T.

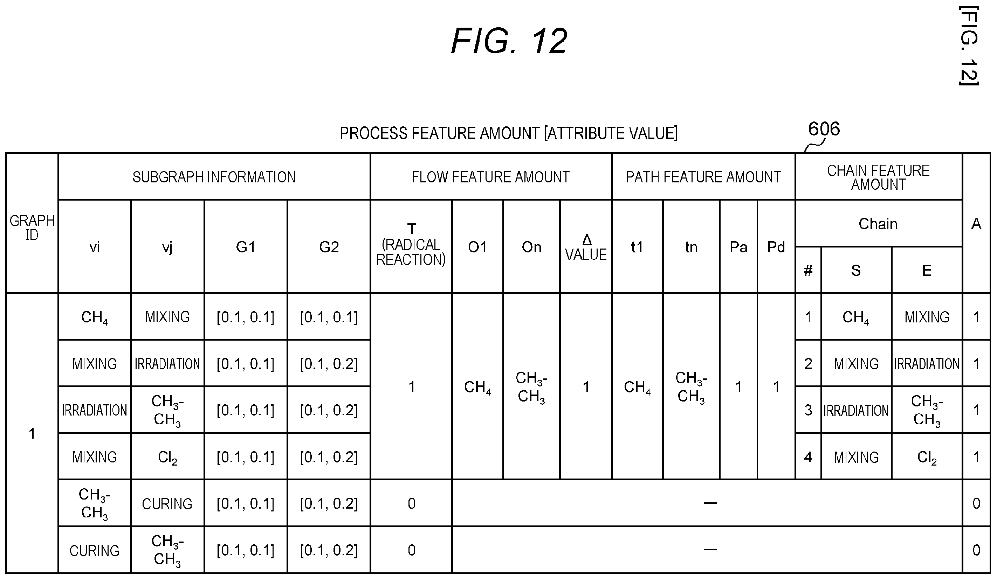

FIG. 12 is a diagram illustrating an example of the process feature amount [attribute value] 606.

The process feature amount [attribute value] 606 includes a graph ID field, a subgraph information field, a flow feature amount field, a path feature amount field, a chain feature amount field, and an A field.

In the graph ID field, a graph ID is registered as an ID of an experimental process.

In the subgraph information field, information regarding each listed edge of the experimental process is registered. The subgraph information field has a vi field, a vj field, a G1 field, and a G2 field. In the vi field, a name of an element corresponding to a node vi at one end of an edge (a node vi in the subgraph G1) is registered. In the vj field, a name of an element corresponding to a node vj at the other end of the edge (a node vj in the subgraph G2) is registered. In the G1 field, a feature amount vector of the subgraph G1 having the node vi is registered. In the G2 field, a feature amount vector of the subgraph G2 having the node vj is registered.

In the flow feature amount field, information regarding a feature amount of a flow of a change from O1 to On due to the application of the treatment (reaction tail) as the frequent process pattern to the experimental process is registered. The flow feature amount field includes a T field, an O1 field, an On field, and a Δ value field. In the T field, a T value indicating whether or not the corresponding treatment (reaction) has been applied to the experimental process is registered. The "corresponding treatment (reaction)" is a radical reaction according to the example illustrated in FIG. 12. The process feature amount [attribute value] 606 may be prepared for each treatment (reaction), and the treatment (reaction) corresponding to the T field may be a treatment (reaction) corresponding to the process feature amount [attribute value] 606. In the O1 field, a name of a substance to which the reaction is to be applied is registered. In the On field, a name of a substance generated as a result of applying the reaction is registered. In the Δ value field, a Δ value is registered as a difference from the t1 value to the tn value.

In the path feature amount field, information regarding a path feature amount indicating whether each listed edge of the experimental process is included in the frequent process pattern is registered. The path feature amount field includes a t1 field, a tn field, a Pa field, and a Pd field. In the t1 field, a name of a source substance t1 to be reacted is registered. In the tn field, a name of a target substance tn generated as a result of the reaction is registered. In the Pa field, a Pa value indicating whether or not the source substance exists in the ancestor of the subgraph G1 is registered. In the Pd field, a Pd value indicating whether or not the target substance exists in the descendant of the subgraph G2 is registered.

In the chain feature amount field, information regarding a chain feature amount indicating which position of the frequent process pattern is matched is registered. The chain feature amount field includes Chain fields, that is, a # field, an S field, and an E field. In the # field, an edge ID of an edge is registered. In the S field, a name of an element corresponding to a node at one end of the edge is registered. In the E field, a name of an element corresponding to a node at the other end of the edge is registered.

In the A field, an A value indicating whether or not an edge exists between a node vi and a node vj in a graph indicating a process as a result of applying the treatment T of the frequent process pattern to the experimental process data is registered. The A value is a binary value indicating whether or not an edge existence probability as an objective variable A is equal to or greater than a threshold.

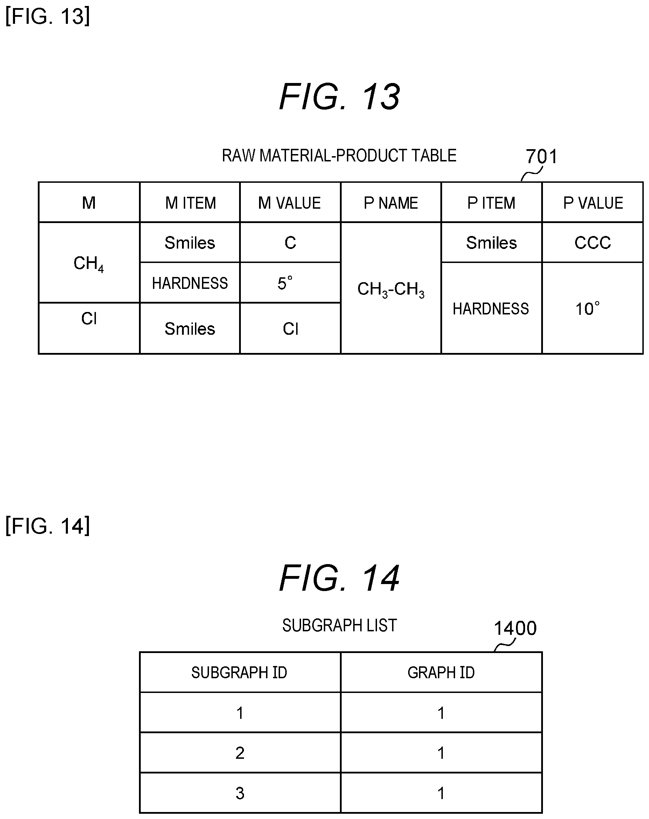

FIG. 13 illustrates an example of the raw material-product table 701.

The raw material-product table 701 has information regarding a combination of one or more raw materials and one or more products. The raw material-product table 701 includes an M field, an M item field, an M value field, a P field, a P item field, and a P value field.

In the M field, a name of a raw material M (substance) is registered. In the M item field, a name of a physical property item of the raw material is registered. In the M value field, an M value, which is a physical property value of the raw material, is registered.

In the P field, a name of a product P (substance) is registered. In the P item field, a name of a physical property item (physical property item) of the product is registered. In the P value field, a P value, which is a physical property value of the product, is registered.

According to the example illustrated in FIG. 13, an experimental process for generating a product CH₃-CH₃ (Smiles "CCC" and hardness "10°") using CH₄ (Smiles "C" and hardness "5°") and Cl (Smiles "Cl") as raw materials is identified in the inference.

FIG. 14 illustrates an example of a subgraph list 1400.

The subgraph list 1400 may be given from the user terminal 101, or may be registered in the structuring support database 130. The subgraph list 1400 includes a subgraph ID field and a graph ID field. In the subgraph ID field, a subgraph ID of a subgraph constructed in the inference processing is registered. In the graph ID field, a graph ID of an experimental process identified from the raw material-product table 701 is registered.

According to the example illustrated in FIG. 14, the graph representing the experimental process identified from the raw material-product table 701 includes three subgraphs.

Hereinafter, an example of processing performed in the present embodiment will be described.

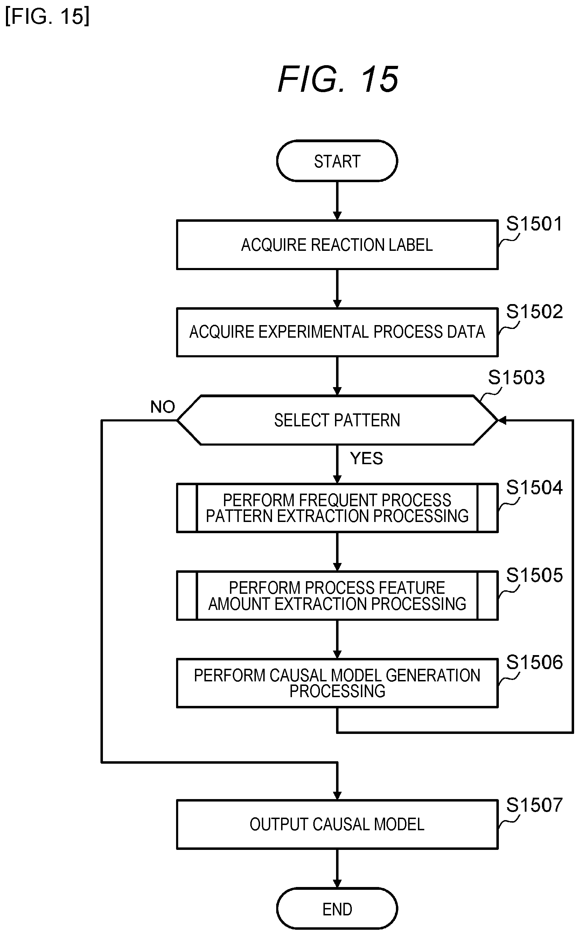

FIG. 15 is a diagram illustrating an example of causal model training processing. The causal model training processing is processing up to outputting a causal model that becomes a final decoder 612 through the causal model generation processing in the training processing illustrated in FIG. 6.

The structuring processing unit 120 acquires a reaction label 602, for example, from the user terminal 101 or the structuring support database 130 (S1501). In addition, the structuring processing unit 120 acquires experimental process data 601 including each graph ID represented by the reaction label 602 from the structured data database 140 (S1502).

When the reaction label 602 acquired in S1501 includes one or more unprocessed pattern names (S1503: YES), the structuring processing unit 120 selects one unprocessed pattern name from among the one or more unprocessed pattern names. The "unprocessed pattern name" is a pattern name that has not yet been selected in the causal model training processing from the acquired reaction label 602. The structuring processing unit 120 performs frequent process pattern extraction processing (S1504), process feature amount extraction processing (S1505), and causal model generation processing (S1506) for the selected unprocessed pattern name. Thereafter, the processing returns to S1503. A flag field may be added to the reaction label 602, and "1" meaning that the treatment (reaction) designated by the selected unprocessed pattern name exists in the graph may be registered in the flag field (see FIG. 8). As a result, in the subsequent processing, it is possible to construct a causal graph in which the subgraph information representing graph structure information for determining whether or not an edge exists, and the flow feature amount, the path feature amount, and the chain feature amount including the treatment (reaction) corresponding to the pattern name are used as explanatory variables and the edge presence probability is used as an objective variable.

When the reaction label 602 acquired in S1501 includes no unprocessed pattern name (S1503: NO), the structuring processing unit 120 outputs a causal model generated for each selected pattern name (S1507). The causal model for each pattern name is used as a final decoder 612.

Note that the causal inference described in WO2023/141019 can be applied to the causal model generation processing (S1506) . That is, A=f(X,Z) is generated as a conditional probability model. A is an objective variable, and represents a probability that an edge exists between the node vi in the subgraph G1 and the node vj in the subgraph G2. An A value (a value indicating whether or not an edge exists) is determined based on this probability (specifically, depending on whether or not this probability is equal to or greater than the threshold). X represents node label feature amounts of the nodes vi and vj and graph feature amounts of G1 and G2 as explanatory variables. Z represents process feature amounts as confounding factors, specifically, a flow feature amount (e.g., a Δ value), a path feature amount (e.g., a Pa value and a Pd value), and a chain feature amount (e.g., an edge ID). Note that the model as the encoder 611 and the model as the decoder 612 that calculate the graph feature amounts may be a graph neural network (GNN), and the edge prediction performed by the decoder 612 using the causal model may be link prediction based on causal inference. The decoder 612 itself may be a conditional probability model. That is, when a graph subject to edge prediction is given as explanatory variables (vi, vj, G1, G2), the structuring processing unit 120 calculates confounding factors (flow feature amount, path feature amount, and chain feature amount) using the frequent process pattern [structure] 603 and the frequent process pattern [attribute value] 604, and calculates an edge existence probability by inputting the confounding factors to the conditional probability model (A=f(X,Z)). The experimental process data input to the encoder 611 includes both the subgraphs G1 and G2 not only in the training processing but also in the inference processing, and since the experimental process data (graph) including both the subgraphs G1 and G2 is used as an input, a probability that G1 and G2 are connected by an edge is calculated. In the frequent process pattern, in an edge corresponding to the edge ID, Chain-S may be node information indicating a start point node (on an upstream side of the process), and Chain-E may be node information indicating an end point node (on a downstream side of the process).

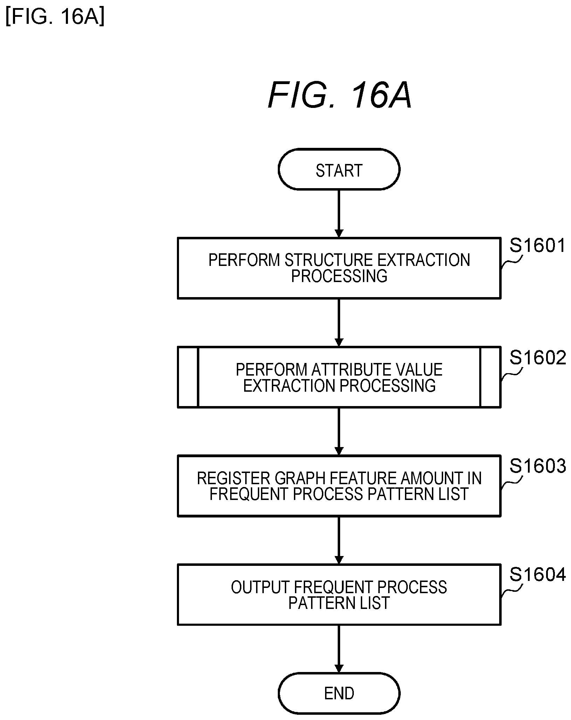

FIG. 16A is a diagram illustrating an example of a flow of frequent process pattern extraction processing (S1504 in FIG. 15) .

The structuring processing unit 120 performs structure extraction processing (S1601). Specifically, the structuring processing unit 120 specifies a common graph portion (common structure) corresponding to the pattern name selected in S1503 as a frequent process pattern in all of the experimental process (graph) represented by all of the experimental process data acquired in S1502, and generates a frequent process pattern [structure] 603 representing the specified frequent process pattern. The frequent process pattern can be specified using GNNExplainer. The GNNExplainer is described, for example, in "YING, Zhitao, et al. Gnnexplainer: Generating explanations for graph neural networks. Advances in neural information processing systems, 2019, 32". Generalization processing may be performed on the experimental process data acquired in S1502 to remove variations in node name notation, and a frequent process pattern may be specified from the experimental process data subjected to the generalization processing. For the generalization processing, for example, the technology described in the present applicant's prior application (Japanese Patent Application No. 2022-200233), which had not been published at the time of filing of the present application, can be utilized.

The structuring processing unit 120 performs attribute value extraction processing (S1602). Through the attribute value extraction processing, a frequent process pattern [structure] 604 is obtained for the pattern name selected in S1503 (the frequent process pattern specified in S1601).

The structuring processing unit 120 registers the extracted graph pattern (frequent process pattern [structure] 604) in the frequent process pattern list (S1603), and outputs the frequent pattern list (S1604). The "frequent process pattern" is a table including a pair of a frequent process pattern [structure] 603 and a frequent process pattern [attribute value] 604, and is used in the inference processing (FIG. 7).

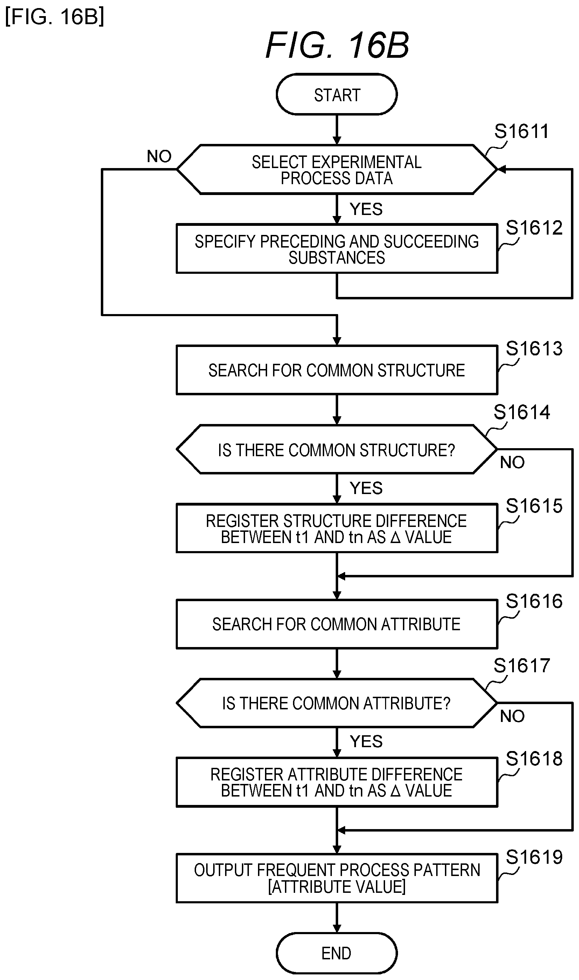

FIG. 16B is a diagram illustrating an example of a flow of attribute value extraction processing.

The structuring processing unit 120 determines whether there is unselected experimental process data among the experimental process data acquired in S1502 (S1611). The "unselected experimental process data" is experimental process data that has not yet been selected in the attribute value extraction processing from among the experimental process data acquired in S1502.

When there is unselected experimental process data (S1611: YES), the structuring processing unit 120 selects the unselected experimental process data, and specifies preceding and succeeding substances of the frequent process pattern specified in S1601 from the experimental process represented by the experimental process data (S1612). In S1612, for the frequent process pattern [attribute value] 604, the graph ID of the selected experimental process data is registered in the graph ID field, the pattern name selected in S1503 is registered in the T field, the names of the preceding and succeeding substances (source substance and target substance) specified in S1612 are registered in the O1 field and the On field, the physical property items and physical property values of the preceding and succeeding substances specified in S1612 are registered in the t1 item field, the t1 value field, the tn item field, and the tn value field, and the edge ID, the name of the start point node, and the name of the end point node are registered in the # field, the S field, and the E field for each edge in the graph portion including the frequent process pattern specified in S1601 and the preceding and succeeding substances specified in S1612. After S1612, the processing returns to S1611.

When there is no unselected experimental process data (S1611: NO), the processing proceeds to S1613.

The structuring processing unit 120 searches for a common structure from the graph portion including the frequent process pattern specified in S1601 and the preceding and succeeding substances specified in S1612 (S1613), and determines whether there is a common structure (S1614). When the t1 item and the tn item are structure items (e.g., "Smiles") and the t1 value and the tn value are structure values, there is a common structure. When there is a common structure (S1614: YES), the structuring processing unit 120 calculates a structure difference between the t1 value and the tn value, and registers the calculated difference as a Δ value in the Δ value field (S1615).

When there is no common structure (S1614: NO), the structuring processing unit 120 searches for a common attribute from the graph portion including the frequent process pattern and the preceding and succeeding substances specified in S1612 (S1616), and determines whether there is a common attribute (S1617). When the t1 item and the tn item are attribute items (e.g., "hardness") and the t1 value and the tn value are attribute values, there is a common attribute. When there is a common attribute (S1617: YES), the structuring processing unit 120 calculates an attribute difference between the t1 value and the tn value, and registers the calculated difference as a Δ value in the Δ value field (S1618).

When there is no common attribute (S1617: NO), or after S1618, the structuring processing unit 120 outputs a frequent process pattern [attribute value] for the treatment (reaction) corresponding to the pattern name selected in S1503 (S1619).

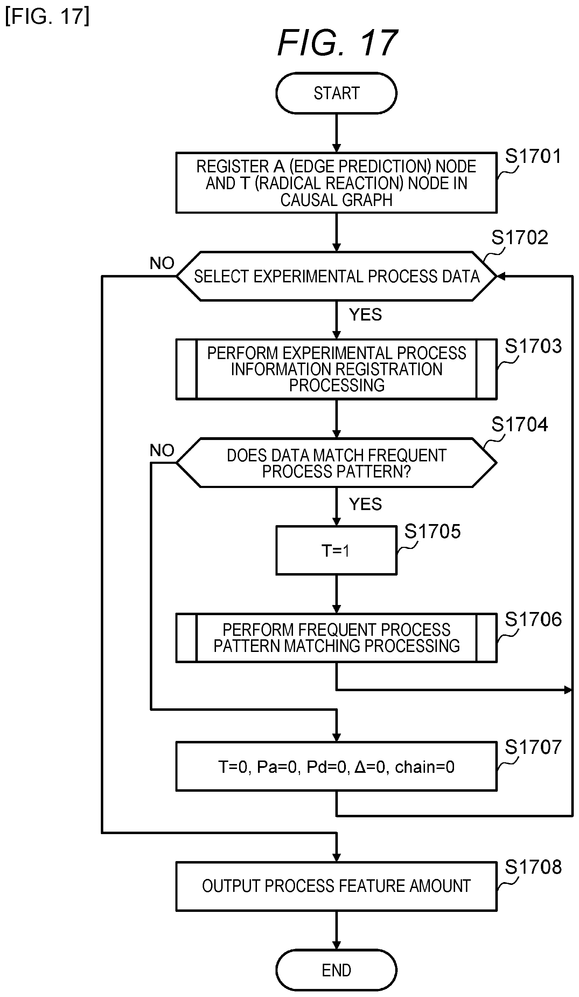

FIG. 17 is a diagram illustrating an example of a flow of process feature amount extraction processing.

The structuring processing unit 120 registers an A (edge prediction) node and a T (radical reaction) node in the process feature amount [structure] 605 (S1701). Note that, in S1701, nodes such as O1 and On (nodes exemplified in FIG. 11), that is, nodes corresponding to the elements registered in the subgraph information field, the flow feature amount field, the path feature amount field, and the chain feature amount field of the process feature amount [attribute value] 606 may be connected to the A node and the T node in the process feature amount [structure] 605. In addition, a blank process feature amount [attribute value] 606 may be prepared.

Next, the structuring processing unit 120 determines whether there is unselected experimental process data among the experimental process data acquired in S1502 (S1702). The "unselected experimental process data" is experimental process data that has not yet been selected in the process feature amount extraction processing from the experimental process data acquired in S1502.

When there is unselected experimental process data (S1702: YES), the structuring processing unit 120 selects the unselected experimental process data, and performs experimental process information registration processing, which is registration processing related to the selected experimental process data (S1703). For the selected experimental process data, the name of the node vi, the name of the node vj, the name of O1, the name of On, the name of the source substance t1, and the name of the target substance tn may be registered in the vi field, the vj field, the O1 field, the On field, the t1 field, and the tn field for each edge in the graph portion including the frequent process pattern specified in S1601 and the preceding and succeeding substances specified in S1612 in the process feature amount [attribute value] 606.

Next, the structuring processing unit 120 determines whether data obtained as a result of the experimental process information registration processing for the selected experimental process data matches the frequent process pattern specified in S1601 (S1704). When the determination result in S1704 is true (S1704: YES), the structuring processing unit 120 registers "1" in the T field of the process feature amount [attribute value] 606 (S1705), and performs frequent process pattern matching processing (S1706). Thereafter, the processing returns to S1702.

When the determination result in S1704 is false (S1704: NO), the structuring processing unit 120 sets "0" (or an invalid value) in the T field, the Pa field, the Pd field, the Δ value field, and the Chain field of the process feature amount [attribute value] 606. Thereafter, the processing returns to S1702.

When there is no unselected experimental process data (S1702: NO), the structuring processing unit 120 outputs a process feature amount [structure] 605 and a process feature amount [attribute value] 606 (S1708).

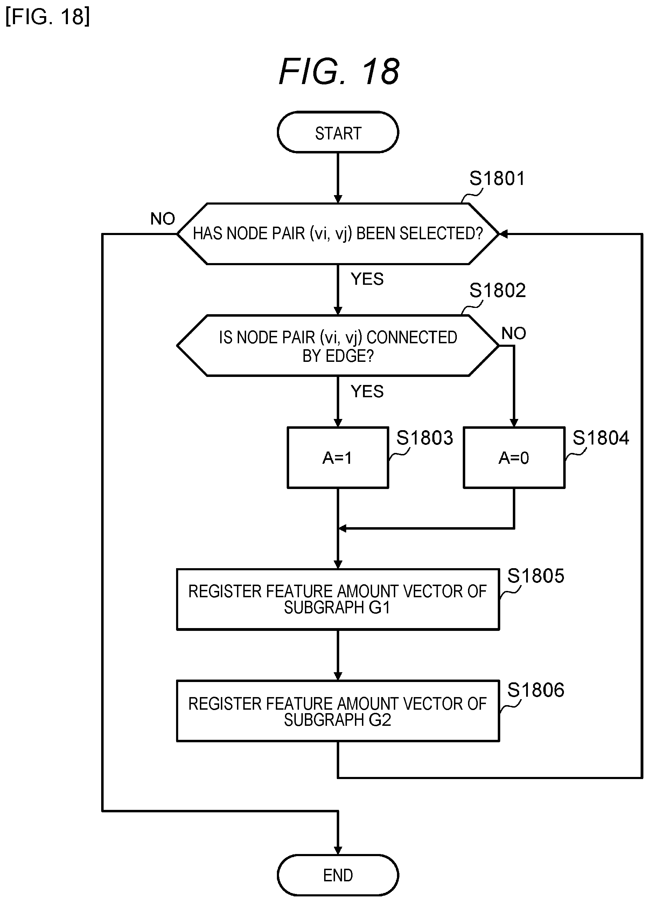

FIG. 18 is a diagram illustrating an example of a flow of experimental process information registration processing.

The structuring processing unit 120 determines whether there is an unselected node pair (vi,vj) in the process feature amount [attribute value] 606 (S1801). The "unselected node pair" is a node pair that has not been selected in the experimental process information registration processing among the node pairs (vi,vj) in the process feature amount [attribute value] 606.

When there is an unselected node pair (vi,vj) (S1801: YES), the structuring processing unit 120 selects the unselected node pair (vi,vj), and determines whether or not the nodes vi and in the selected node pair are connected by an edge (S1802). When the determination result in S1802 is true (S1802: YES), the structuring processing unit 120 registers an A value of the node pair (vi,vj) as "1" in the A field (S1803). When the determination result in S1802 is false (S1802: NO), the structuring processing unit 120 registers an A value of the node pair (vi,vj) as "0" in the A field (S1804).

After S1803 or S1804, for the node pair (vi,vj), the structuring processing unit 120 registers a feature amount vector of the subgraph G1 having the node vi in the G1 field (S1805), and registers a feature amount vector of the subgraph G2 having the node vj in the G2 field (S1806).

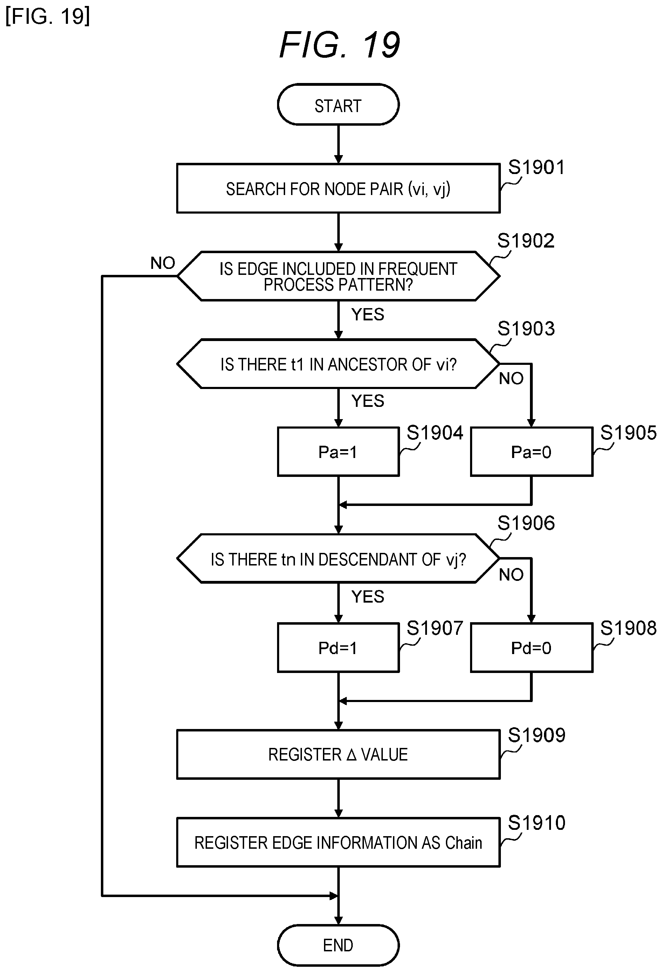

FIG. 19 is a diagram illustrating an example of a flow of frequent process pattern matching processing.

The structuring processing unit 120 searches for a node pair (vi,vj) in the frequent process pattern (S1901), and determines whether an edge in a found node pair (vi,vj) is included in the frequent process pattern (S1902).

When the determination result in S1902 is true (S1902: YES), the structuring processing unit 120 determines whether the ancestor node of the node vi in the found node pair (vi,vj) is a node O1 (S1903). When the determination result in S1903 is true (S1903: YES), the structuring processing unit 120 registers a Pa value of the node pair (vi,vj) as "1" in the Pa field (S1904). When the determination result in S1903 is false (S1903: NO), the structuring processing unit 120 registers a Pa value of the node pair (vi,vj) as "0" in the Pa field (S1905) .

After S1904 or S1905, the structuring processing unit 120 determines whether or not the descendant node of the node vj in the found node pair (vi,vj) is a node On (S1906). When the determination result in S1906 is true (S1906: YES), the structuring processing unit 120 registers a Pd value of the node pair (vi,vj) as "1" in the Pd field (S1907). When the determination result in S1906 is false (S1906: NO), the structuring processing unit 120 registers a Pd value of the node pair (vi,vj) as "0" in the Pd field (S1908).

After S1907 or S1908, the structuring processing unit 120 registers, as a Δ value, a value as a difference between the physical property value of t1 and the physical property value of tn in the Δ value field (S1909). The structuring processing unit 120 registers an edge ID, a node name of a start point node, and a node name of an end point node in the # field, the S field, and the E field as edge information of the frequent process pattern (S1910).

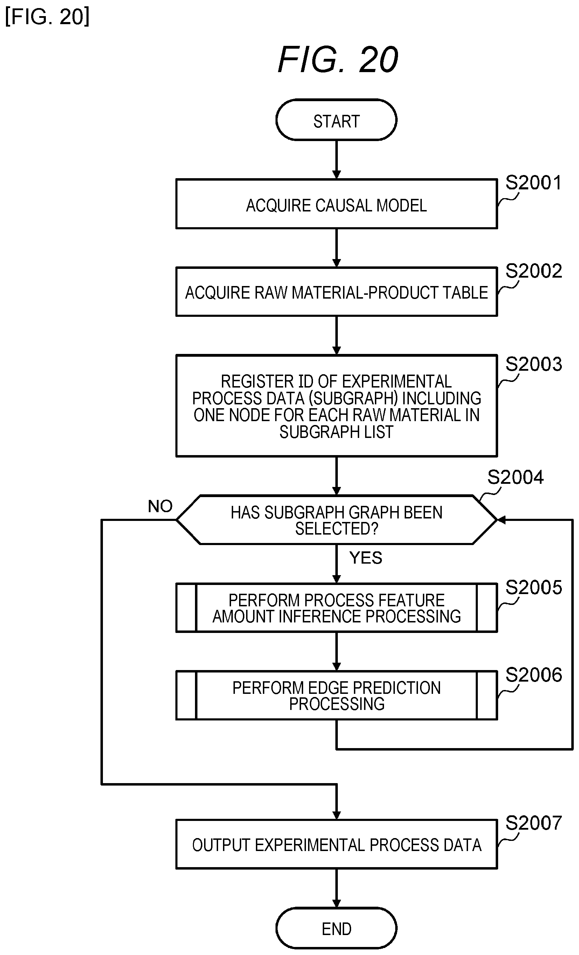

FIG. 20 is a diagram illustrating an example of a flow of graph construction processing.

The structuring processing unit 120 acquires a causal model generated in the causal model generation processing of the training processing (S2001). The causal model may be present for each treatment (reaction).

The structuring processing unit 120 acquires the raw material-product table 701, for example, from the user terminal 101 or the structuring support database 130 (S2002).

For each raw material represented by the M field in the raw material-product table 701 acquired in S2002, the structuring processing unit 120 generates experimental process data (subgraph) 702 including one node corresponding to the raw material, and registers the subgraph ID of the experimental process data in the subgraph list 1400 (S2003). The subgraph list 1400 may be stored in the structuring support database 130.

The structuring processing unit 120 determines whether there are two or more unselected subgraphs in the subgraph list 1400 (S2004). The "unselected subgraph" is a subgraph corresponding to the subgraph ID that has not been selected in the graph construction processing among the subgraph IDs in the subgraph list 1400.

When there are two or more unselected subgraphs (S2004: YES), the structuring processing unit 120 selects one unselected subgraph and performs process feature amount inference processing (S2005) and edge prediction processing (S2006). The structuring processing unit 120 updates the subgraph based on the edge prediction result, and registers the subgraph ID of the updated subgraph in the subgraph list 1400. As a result, the number of unselected subgraphs increases. Thereafter, the processing returns to S2004.

When there are no two or more unselected subgraphs (S2004: NO), the subgraph list 1400 includes an ID of one unselected subgraph. One unselected subgraph is a subgraph expanded as a result of inductive inference in the graph construction processing (one graph constructed as a result of connecting two or more subgraphs using an edge). The structuring processing unit 120 outputs structured data representing the subgraph (graph) as experimental process data (S2007).

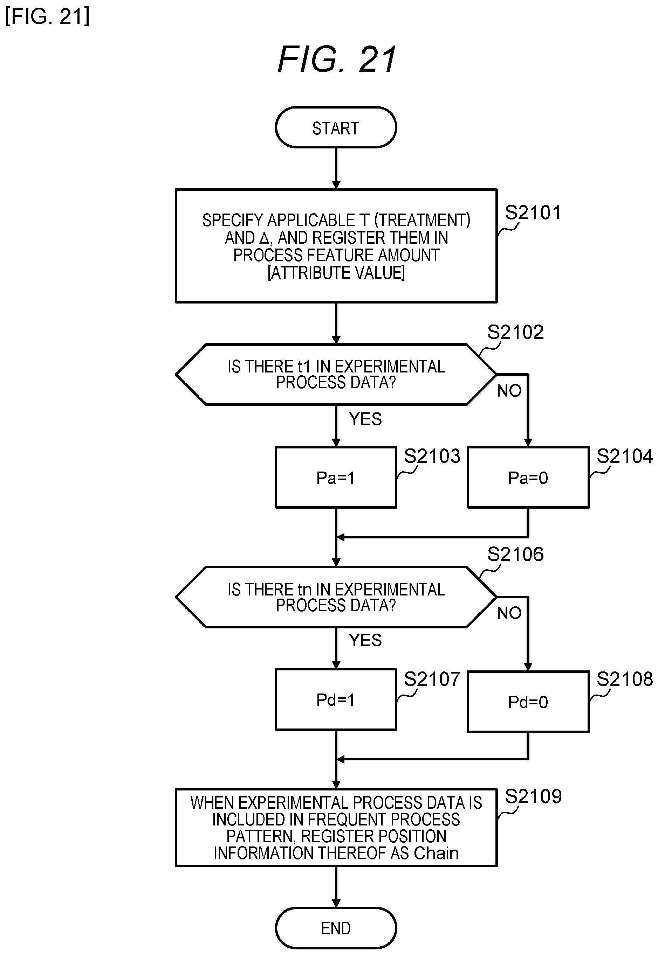

FIG. 21 is a diagram illustrating an example of a flow of process feature amount inference processing.

The structuring processing unit 120 compares the M field (raw material name) and the P field (product name) of the raw material-product table 701 acquired in S2002 with the source substance name (O1 value) and the target substance name (On value) specified from the frequent process pattern [attribute value] 604 output in the training processing, specifies a treatment T that is likely to be provided and an application condition Δ value thereof, and registers the value of the treatment T and the Δ value in the T field and the Δ value field of the process feature amount [attribute value] 706 (S2101). The "treatment T that is likely to be provided" is a treatment (reaction) corresponding to a pair of a source substance name and a target substance name that matches a pair of a raw material name and a product name, and "1" is registered in the T field as a value of such treatment T. The "application condition Δ value" is a Δ value corresponding to a pair of a physical property value of the source substance and a physical property value of the target substance and registered in the frequent process pattern [attribute value] 604. In the process feature amount [attribute value] 706, for a pair of the subgraph G1 selected in S2004 and each subgraph G2 other than the subgraph G1, a node name of a node vi (e.g., a node corresponding to a raw material), a node name of a node vj (e.g., a node corresponding to a product), a feature amount vector of the subgraph G1, a feature amount vector of the subgraph G2, O1 (raw material name), On (product name), t1 (raw material name), and tn (product name) may be registered in the vi field, the vj field, the G1 field, the G2 field, the O1 field, the On field, the t1 field, and the tn field of the process feature amount [attribute value] 706.

Next, the structuring processing unit 120 determines whether the node corresponding to the raw material t1 represented by the raw material-product table 701 is included in the experimental process data (subgraph) 702 (S2102). When the determination result in S2102 is true (S2102: YES), the structuring processing unit 120 registers a Pa value corresponding to the pair of the raw material t1 and the product tn as "1" in the Pa field (S2103). When the determination result in S2102 is false (S2102: NO), the structuring processing unit 120 registers a Pa value corresponding to the pair of the raw material t1 and the product tn as "0" in the Pa field (S2104) .

After S2103 or S2104, the structuring processing unit 120 determines whether the node corresponding to the product tn represented by the raw material-product table 701 is included in the experimental process data (subgraph) 702 (S2106). When the determination result in S2106 is true (S2106: YES), the structuring processing unit 120 registers a Pd value corresponding to the pair of the raw material t1 and the product tn as "1" in the Pd field (S2107) . When the determination result in S2106 is false (S2106: NO), the structuring processing unit 120 registers a Pd value corresponding to the pair of the raw material t1 and the product tn as "0" in the Pd field (S2108).

After S2107 or S2108, when the frequent process pattern [structure] 603 obtained in the training processing is included in the experimental process data (subgraph) 702, the structuring processing unit 120 registers position information (edge ID, start point node name, and end point node name) for each edge in the Chain field of the process feature amount [attribute value] 706 based on the frequent process pattern [attribute value] 604 corresponding to the frequent process pattern [structure] 603 in the frequent process pattern (S2109).

In the process feature amount inference processing, a process feature amount [attribute value] 706 is generated. As described above, the process feature amount [structure] 605 is data generated by the training processing.

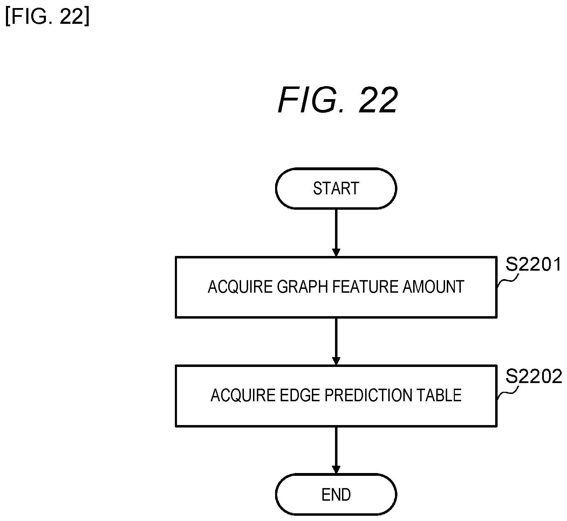

FIG. 22 is a diagram illustrating an example of a flow of edge prediction processing.

The structuring processing unit 120 acquires a graph feature amount from the encoder 611 by inputting experimental process data (subgraph) 702 to the encoder 611 trained in the training processing (S2201).

The structuring processing unit 120 acquires an edge prediction table 706 from the decoder 612 by inputting the graph feature amount generated in S2201 and the values (explanatory variables (vi, vj, G1, and G2) and confounding factors) obtained from the process feature amount [structure] 605 generated in the training processing and the process feature amount [attribute value] 706 generated in the process feature amount inference processing to the decoder 612 (the causal model [E=f(ZG, process feature amount)]) trained in the training processing (S2202). The edge prediction table 706 represents a binary value indicating whether or not a subgraph G1 (a subgraph including a node corresponding to a raw material) as experimental process data (subgraph) 702 and another subgraph G2 (a subgraph including a node corresponding to a product) are connected by an edge.

The above processing will be explained again, roughly divided into training processing and inference processing, for example, as follows.

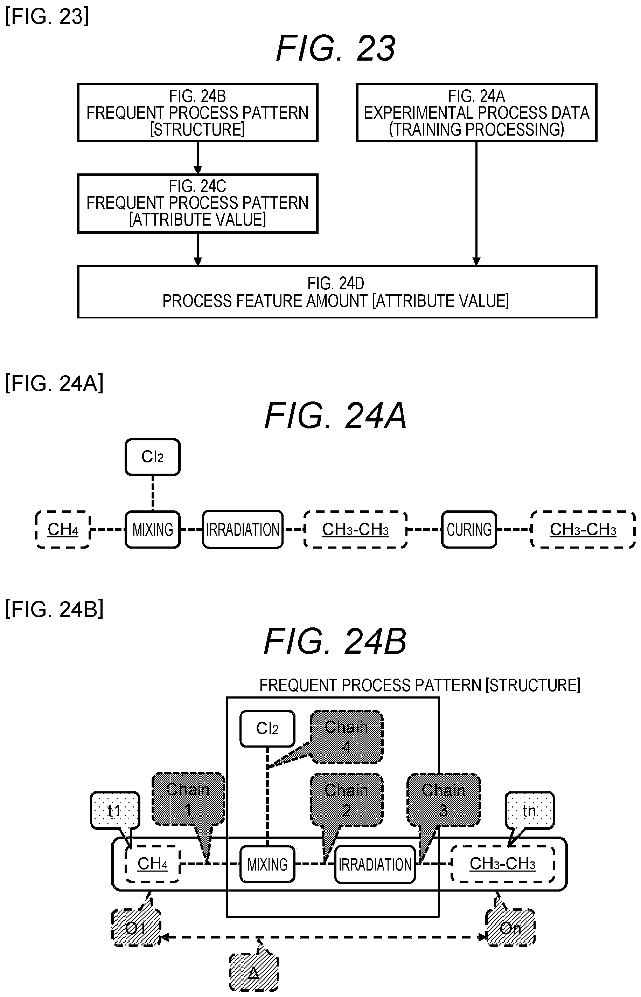

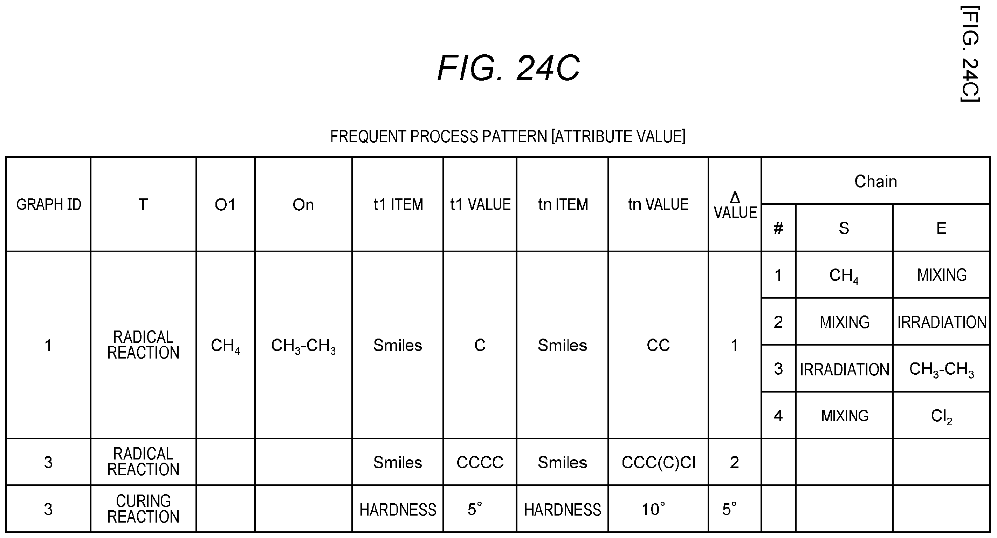

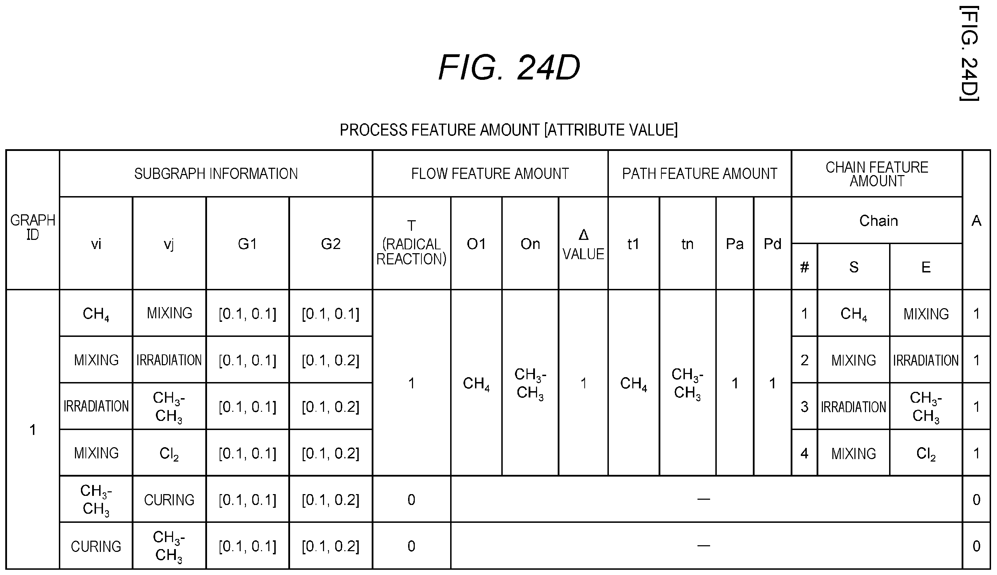

FIG. 23 is a diagram schematically illustrating an example of a part of training processing. FIG. 24A illustrates an example of experimental process data selected in this example. FIG. 24B illustrates a frequent process pattern [structure] representing a frequent process pattern (radical reaction) extracted in this example in a part of an experimental process represented by the experimental process data illustrated in FIG. 24A. FIG. 24C illustrates a frequent process pattern [attribute value] generated in this example. FIG. 24D illustrates a process feature amount [attribute value] generated in this example.

The frequent process pattern [structure] corresponds to a record having a graph ID "1" in the frequent process pattern [attribute value]. The preceding and succeeding substances of the frequent process pattern (radical reaction) are O1 and t1 (CH₄) and On and tn (CH₃-CH₃).

In addition, each edge of the experimental process data and each record of the process feature amount [attribute value] correspond to each other. The first to fourth records of the process feature amount [attribute value] correspond to four edges in a graph portion (a graph portion illustrated in FIG. 24B) including the frequent process pattern (radical reaction) and the preceding and succeeding substances thereof. Chain1 to Chain4 illustrated in FIG. 24B mean chain feature amounts of the four edges.

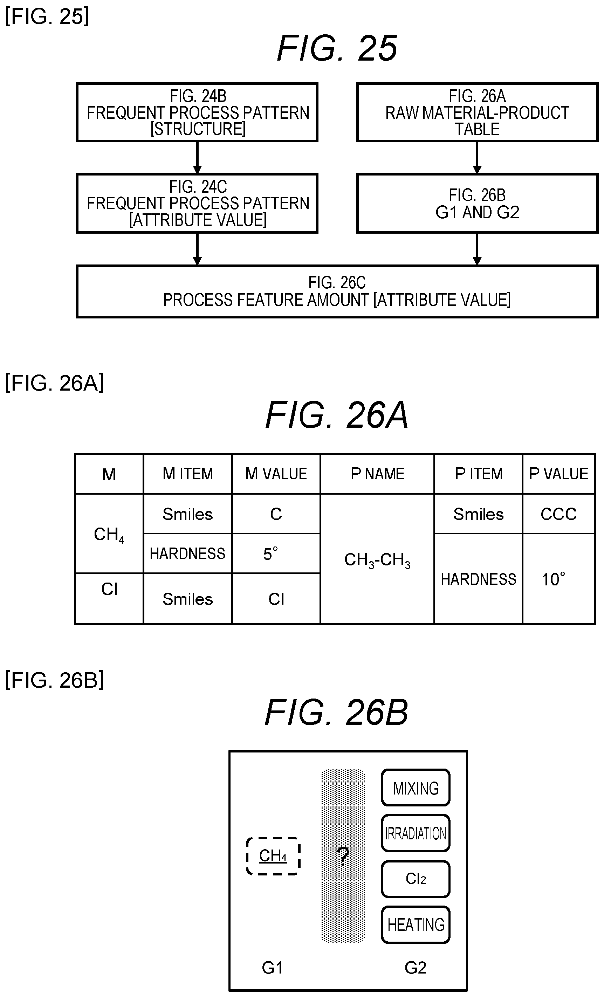

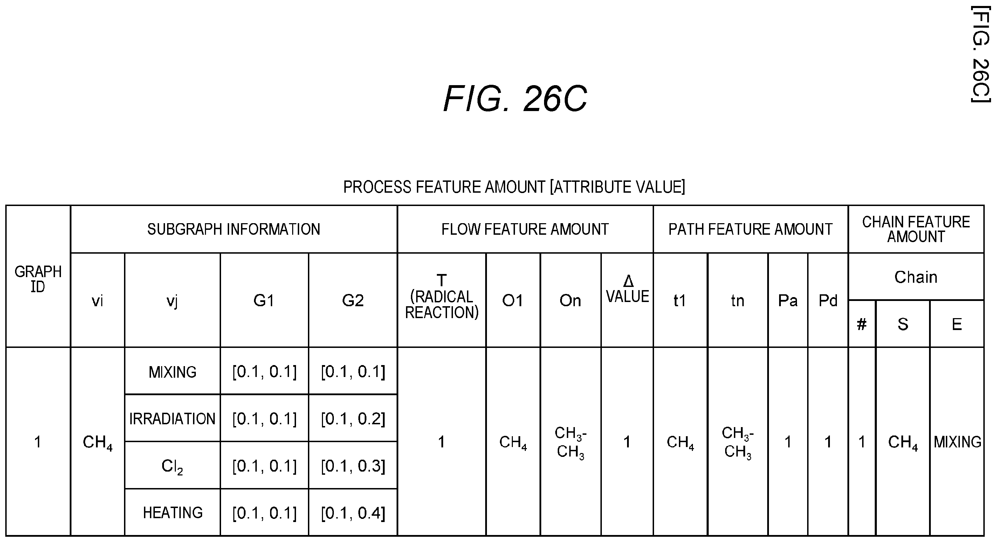

FIG. 25 is a diagram schematically illustrating an example of a part of inference processing. FIG. 26A is a diagram illustrating an example of a raw material-product table provided in this example. FIG. 26B is a diagram illustrating an example of a subgraph G1 and a subgraph G2 determined from the raw material-product table. FIG. 26C illustrates a process feature amount [attribute value] generated in this example. The frequent process pattern [structure] and the frequent process pattern [attribute value] used in the inference processing according to this example are data generated by the training processing. Candidates for the subgraph G2 may include (1) candidates for operation nodes (assumed to be finite and enumerated in advance), (2) substance nodes (e.g., Cl₂ in the example of FIG. 9) appearing in the frequent process pattern, or (3) substances of products (P names) in the raw material-product table 701.

In this example, one node corresponding to the raw material (CH₄) represented by the raw material-product table is the subgraph G1, and one node for each element (e.g., a substance or an operation) in the frequent process pattern [structure] obtained in the training processing is the subgraph G2. In inductive inference, a next node (subgraph G2) of the subgraph G1 (initially, one CH₄ node according to the example illustrated in FIG. 26B) is predicted, and the subgraph G1 is expanded by connecting any subgraph G2 to the subgraph G1 using an edge. Such processing is repeated, and eventually, experimental process data representing a predicted experimental process for generating a product from a raw material is obtained.

Each record of the process feature amount [attribute value] exemplified in FIG. 26C corresponds to a respective one of four nodes (four subgraphs G2) that are likely to be connected to the subgraph G1 (CH₄ node).

In addition, since it can be seen that the M name and the P name in the raw material-product table match the preceding and succeeding substances of the frequent process pattern of the radical reaction, the structuring processing unit 120 registers "1" as a T value, a Pa value, and a Pd value in the T field, the Pa field, and the Pd field of the process feature amount [attribute value] exemplified in FIG. 26C, respectively.

In addition, as a chain feature amount, the structuring processing unit 120 sets Chain1 corresponding to an edge in which a source substance (a substance corresponding to the start point node) of the frequent process pattern is CH₄ for all of the four records. That is, the node with the highest likelihood among the four records is predicted using Chain1. In this case, by searching the Chain-S field of the frequent process pattern [attribute value] 604 for the starting point node of G1 ("CH₄" in this case), it is possible to specify, before edge prediction, that "mixing" becomes a candidate after edge ID=1. In a case where "CH₄" exists multiple times in the Chain-S field, by setting edge IDs from the side closer to the start point node, it is possible to uniquely set candidates in order from the side from the smaller edge ID.

### Second Embodiment

In the second embodiment, measurement process data is generated instead of the experimental process data described in the first embodiment. In the description of the present embodiment, differences from the first embodiment will be mainly described, and descriptions of common points with the first embodiment will be omitted or simplified.

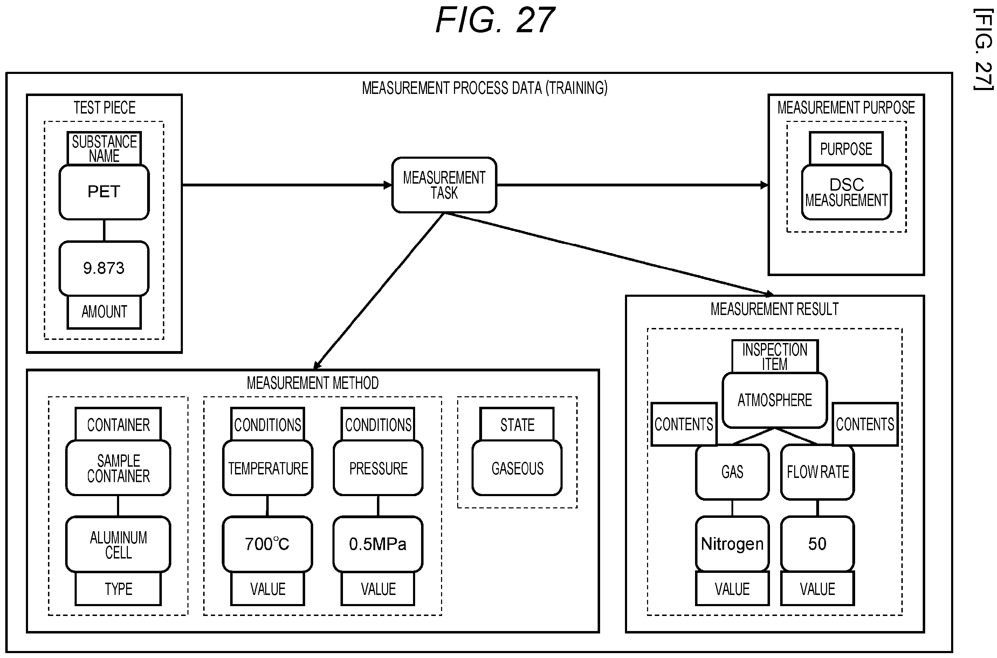

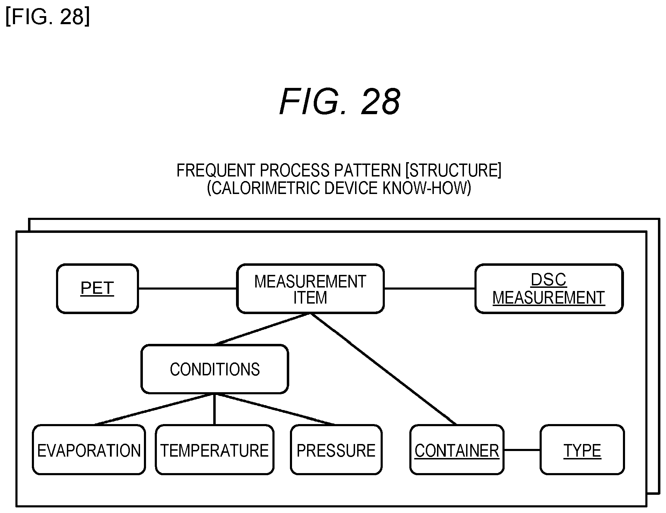

FIG. 27 is a diagram illustrating an example of measurement process data according to the second embodiment. FIG. 28 is a diagram illustrating an example of a frequent process pattern [structure] according to the second embodiment.

The second embodiment is applied to a task of measuring performance of a sample (e.g., a product). For example, a measurement department measures whether target performance is achieved by subjecting a test piece of a sample to a measurement device, and creates a measurement result report. In general, the contents of the report vary depending on the person in charge, and there are cases where specifications (details) such as the type (e.g., the material of the container in which the test piece of the sample is placed) of experimental equipment used are not included. When this report is reused as know-how in another measurement task, if the type of the experimental equipment that has been omitted can be predicted, reusability is improved, and work efficiency is expected to increase. In the second embodiment, it is possible to predict the type of experimental equipment in the measurement process.

The frequent process pattern [structure] is structured data representing a frequent process pattern as a partial structure common between the measurement processes. The frequent process pattern exemplified in FIG. 28 is a know-how pattern indicating that "in a DSC measurement task for PET (polyethylene resin), when the type of the container in which the test piece is placed is selected, it is determined based on conditions such as whether evaporation occurs, temperature, and pressure". DSC stands for differential scanning calorimetry.

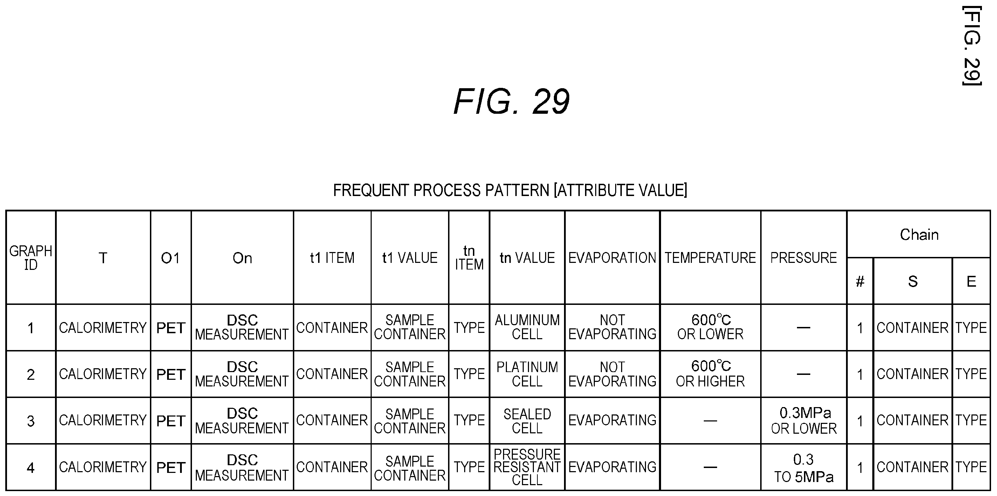

FIG. 29 is a diagram illustrating an example of a frequent process pattern [attribute value] according to the second embodiment.

The frequent process pattern [attribute value] according to the second embodiment includes, instead of the Δ value field, an evaporation field, a temperature field, and a pressure field in which whether evaporation occurs, a temperature value, and a pressure value are registered as conditions for selecting tn. The graph ID is an ID of a measurement process. In the first embodiment, O1 and t1 are the same substance, and On and tn are the same substance. However, in the second embodiment, O1 and t1 are different, and On and tn are different. Specifically, in the second embodiment, O1 represents a substance, On represents a measurement method, t1 represents a container, and tn represents a type of the container.

For example, according to the first record, it can be seen that in the measurement process corresponding to the graph ID "1", an aluminum cell was selected as a sample container type when performing DSC measurement on PET by applying calorimetric device know-how because the target PET does not evaporate and the temperature is 600°C or lower.

Note that the frequent process pattern [structure] and the frequent process pattern [attribute value] exemplified in FIGS. 28 and 29 may be input from the user via the user terminal 101.

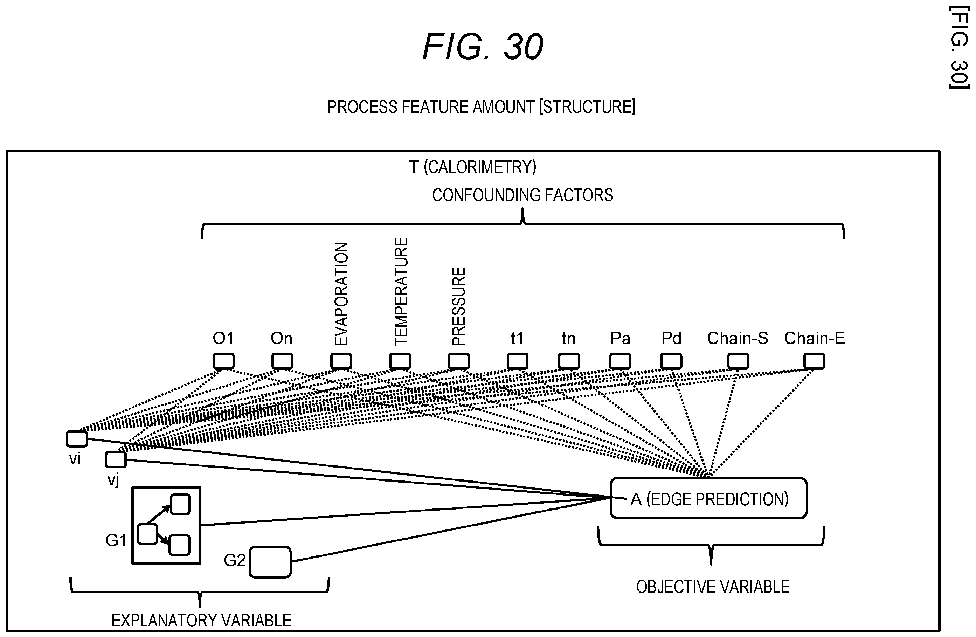

FIG. 30 is a diagram illustrating an example of a process feature amount [structure] according to the second embodiment.

In the process feature amount [structure] according to the second embodiment, the explanatory variable is an explanatory variable (e.g., a treatment such as calorimetry) extracted from the measurement process. The objective variable is an edge existence probability as in the first embodiment.

The example illustrated in FIG. 30 is, for example, as follows.
- A (edge existence probability) in a case where T (calorimetry know-how) is applied between the node vi in the subgraph G1 and the node vj in the subgraph G2 of the measurement process is predicted.
- In the sample O1 and the purpose On, it is indicated whether the evaporation, the temperature, and the pressure meet the conditions.
- The Pa value represents whether a subgraph G1 exists on a path between a prediction target t1 and a prediction result tn, that is, whether the prediction target t1 exists in the ancestor.
- The Pd value represents whether a subgraph G2 exists on a path between the prediction target t1 and the prediction result tn, that is, whether the prediction result tn exists in the descendant.
- Chain represents positions where the subgraph G1 and the subgraph G2 exist in the frequent process pattern of the treatment T.

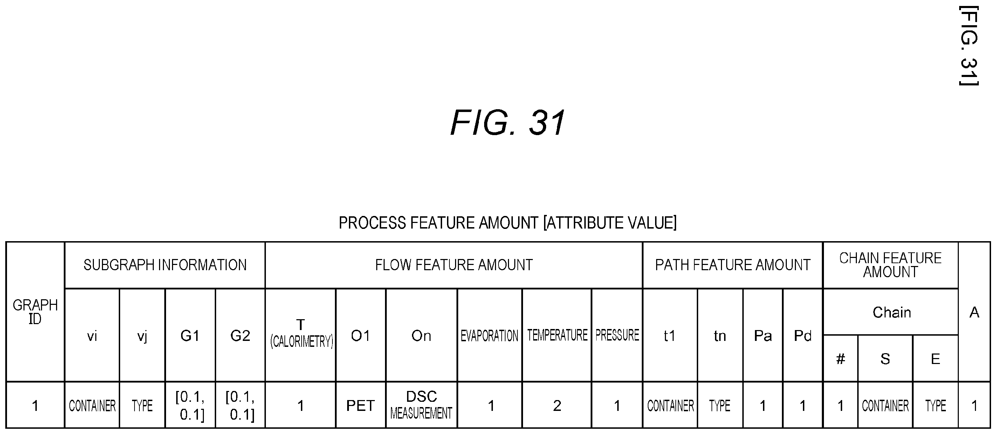

FIG. 31 is a diagram illustrating an example of a process feature amount [attribute value] according to the second embodiment.

The process feature amount [attribute value] according to the second embodiment includes, instead of the Δ value, an evaporation field, a temperature field, and a pressure field, similarly to the frequent process pattern [attribute value].

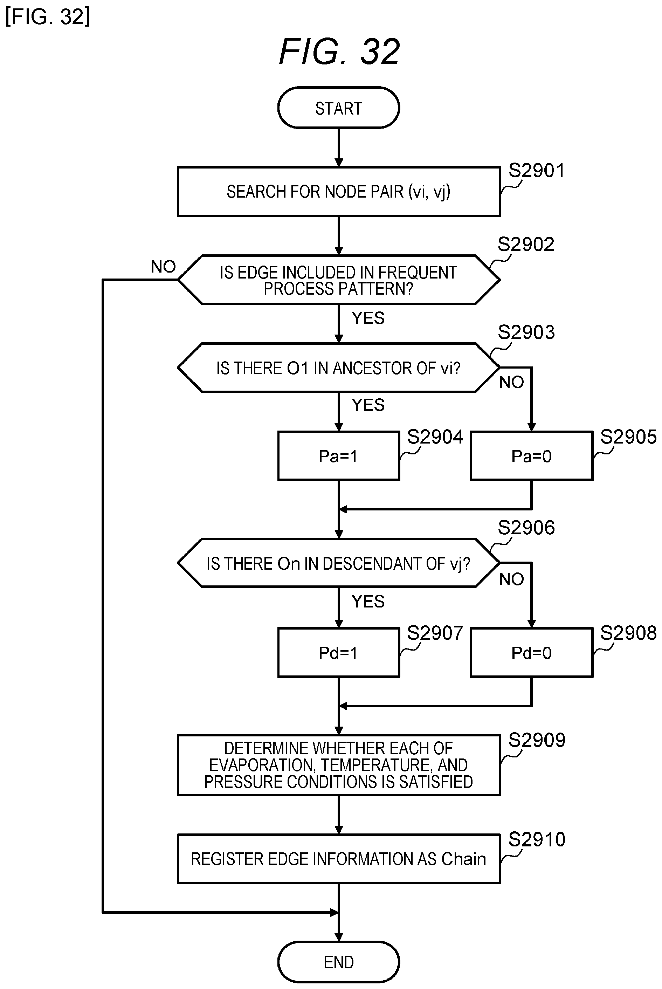

FIG. 32 is a diagram illustrating an example of a flow of frequent process pattern matching processing according to the second embodiment.

The structuring processing unit 120 searches for a node pair (vi,vj) in the frequent process pattern (S2901), and determines whether an edge in a found node pair (vi,vj) is included in the frequent process pattern (S2902).

When the determination result in S2902 is true (S2902: YES), the structuring processing unit 120 determines whether the ancestor node of the node vi in the found node pair (vi, vj) is a node O1 (S2903). When the determination result in S2903 is true (S2903: YES), the structuring processing unit 120 registers a Pa value of the node pair (vi,vj) as "1" in the Pa field (S2904). When the determination result in S2903 is false (S2903: NO), the structuring processing unit 120 registers a Pa value of the node pair (vi,vj) as "0" in the Pa field (S2905) .

After S2904 or S2905, the structuring processing unit 120 determines whether or not the descendant node of the node vj in the found node pair (vi,vj) is a node On (S2906) . When the determination result in S2906 is true (S2906: YES), the structuring processing unit 120 registers a Pd value of the node pair (vi,vj) as "1" in the Pd field (S2907) . When the determination result in S2906 is false (S2906: NO), the structuring processing unit 120 registers a Pd value of the node pair (vi,vj) as "0" in the Pd field (S2908).

After S2907 or S2908, the structuring processing unit 120 registers whether evaporation occurs, a temperature value, and a pressure value in the evaporation field, the temperature field, and the pressure field of the process feature amount [attribute value] (S2909). Whether evaporation occurs, the temperature value, and the pressure value may be values calculated by comparing respective values of the measurement process data with the conditions of the frequent process pattern [attribute value]. Since the values in the "evaporation", "temperature", and "pressure" fields of the process pattern are conditions, the structuring processing unit 120 determines whether the respective values of the measurement process data satisfy the conditions in S2909. For example, according to the measurement process data (at the time of training) exemplified in FIG. 27, since the "state is gaseous", evaporation occurs, and therefore, "1" may be registered for the evaporation. Since the "temperature is 700°C", "2" may be registered as the temperature of 600°C or higher (the upper limit or higher). Since the "pressure is 0.5 MPa", "1" may be registered for the pressure of 0.3 to 5 MPa (the lower limit or more and the upper limit or less) . That is, the value for evaporation may be either "0" (not evaporating) or "1" (evaporating), and the values for temperature and pressure may be any of "0" (the lower limit value or less), "1" (between the upper limit value and the lower limit value), and "2" (the upper limit value or more).

The structuring processing unit 120 registers an edge ID, a node name of a start point node, and a node name of an end point node in the # field, the S field, and the E field as edge information of the frequent process pattern (S2910).

According to the first embodiment and the second embodiment described above, for example, the following applies.

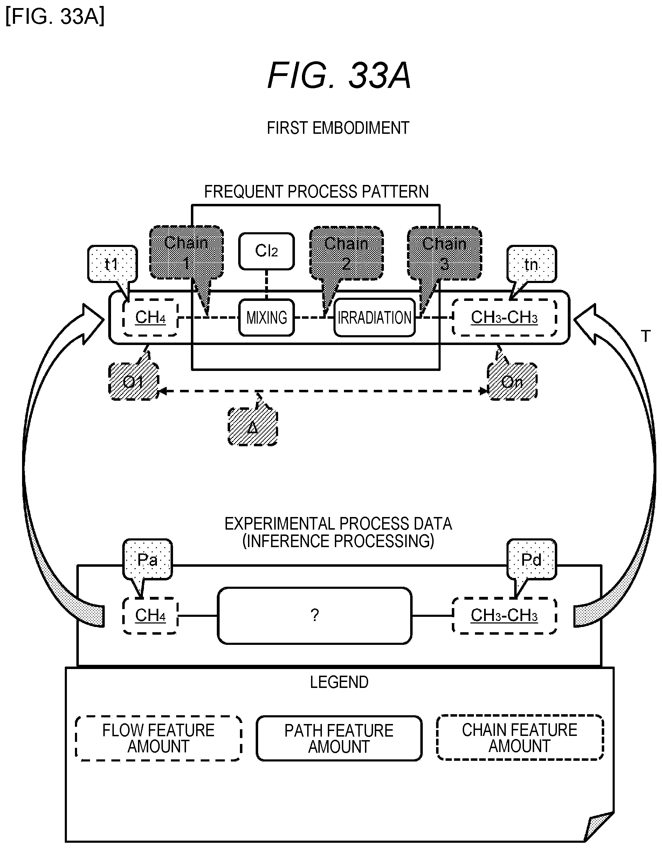

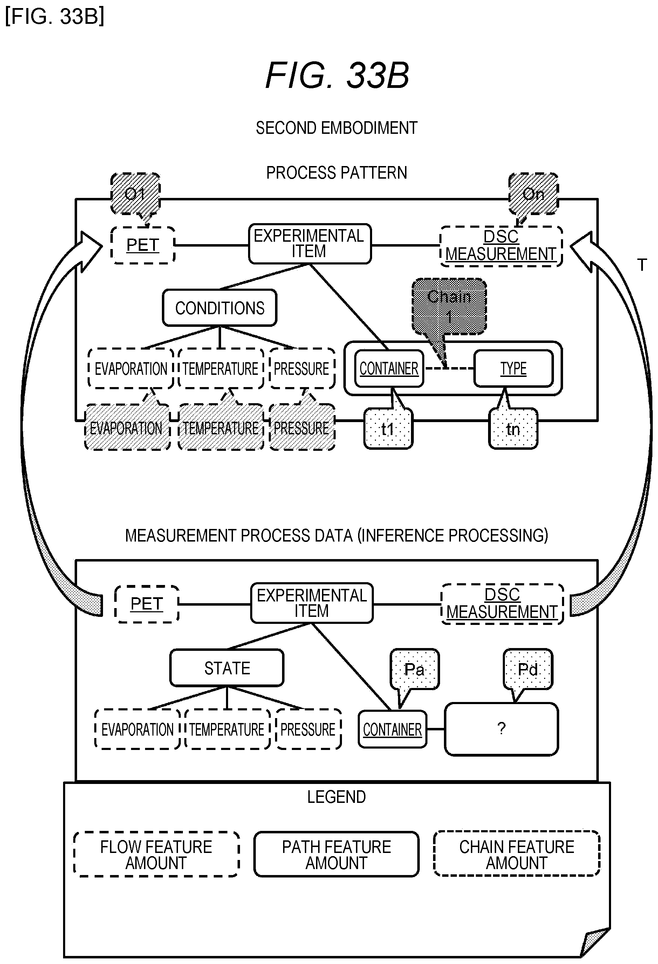

FIG. 33A is a diagram schematically illustrating an example of matching (matching between a frequent process pattern and experimental process data) according to the first embodiment. FIG. 33B is a diagram schematically illustrating an example of matching (matching between a frequent process pattern and measurement process data) according to the second embodiment.

In an example of an experimental process according to the first embodiment, in order to predict a path from a raw material to a product, O1/On, which represent a reaction purpose for causing a chemical reaction, and t1/tn, which represent both ends of the path predicted using a frequent process pattern, are identical. On the other hand, in an example of a measurement process according to the second embodiment, information other than the predicted path is included in the frequent process pattern, and O1/On and t1/tn are different.

In addition, in the first embodiment, the flow feature amount may include a Δ value as a difference between a physical property value of t1 and a physical property value of tn, and in the second embodiment, the flow feature amount may include respective thresholds of whether evaporation occurs, a temperature value, and a pressure value.

As described above, according to the first embodiment and the second embodiment, it is possible to construct a causal model with less training data and pattern description by using the frequent process pattern and the process feature amount. Furthermore, by modeling process determining factors, the unknown process prediction accuracy is improved.

It should be noted that the present invention is not limited to the above-described embodiments, and includes various modifications. In addition, for example, the configurations of the above-described embodiments have been described in detail in order to explain the present invention in an easy-to-understand manner, and the present invention is not necessarily limited to having all the configurations described above. In addition, other configurations may be added to some of the configurations of each embodiment, some of the configurations of each embodiment may be deleted, or some of the configurations of each embodiment may be replaced with other configurations.

In addition, each of the above-described configurations, functions, processing units, processing means, and the like may be realized in hardware, for example, by designing some or all thereof as an integrated circuit. In addition, the present invention can also be implemented by a program code of software that realizes the functions of the embodiments. In this case, a storage medium in which the program code is recorded is provided to a computer, and a processor included in the computer reads the program code stored in the storage medium. In this case, the program code itself read from the storage medium realizes the functions of the above-described embodiments, and the program code itself and the storage medium storing the program code constitute the present invention. As a storage medium for supplying such a program code, for example, a flexible disk, a CD-ROM, a DVD-ROM, a hard disk, a solid state drive (SSD), an optical disk, a magneto-optical disk, a CD-R, a magnetic tape, a non-volatile memory card, a ROM, or the like is used.

In addition, the program code realizing the functions described in the present embodiments can be implemented by a wide range of programs or script languages, for example, assembler, C/C++, perl, Shell, PHP, Python, and Java (registered trademark).

Furthermore, by distributing the program code of software that realizes the functions of the embodiments via a network, the program code may be stored in a storage means such as a hard disk or a memory of the computer or in a storage medium such as a CD-RW or a CD-R, and the processor included in the computer may read and execute the program code stored in the storage means or the storage medium.

In addition, in the above-described embodiments, the control lines and information lines are those that are considered necessary for the description, and all the control lines and information lines on the product are not necessarily shown. All the configurations may be connected to each other.

The above description can be summarized, for example, as follows. The following summary may include the above supplementary description and description of modifications.

The structuring processing apparatus 100 outputs structured data of a process in which a prediction target is predicted based on a process feature amount that is a feature amount based on a coarse granularity such as a process pattern of a treatment applied to the process, rather than a fine granularity such as elements in the process having a plurality of elements including a plurality of procedures. As a result, the number of combinations is reduced, and external factors of the elements are also taken into consideration in the prediction, thereby making it possible to reduce a computational load of processing for predicting a process represented by generated structured data and improve process prediction accuracy.

The structuring processing apparatus (e.g., the structuring processing apparatus 100) includes a storage device (e.g., the main storage device 202 and the auxiliary storage device 203) and an arithmetic device (e.g., the arithmetic device 201). The storage device stores treatment process pattern structured data (e.g., a frequent process pattern [structure] 603) representing a graph of a treatment process pattern (e.g., a frequent process pattern) for each of one or more treatments and treatment process pattern attribute value data (e.g., a frequent process pattern [attribute value] 604) that is data including an attribute value related to an element represented by the treatment process pattern structured data. For each of the one or more treatments, the treatment process pattern is a process portion having a structure common to two or more processes. For each of the one or more treatments, the treatment process pattern attribute value data includes attribute values related to a purpose element that is an element as a purpose for which the treatment is applied, a result element that is an element as a result of applying the treatment, a source element that is an element to which the treatment is applied, and a target element that is an element when the treatment is applied to the source element. The arithmetic device performs the following operations (A) to (F).
(A) In a process including a purpose element, a result element, and a source element and of which a path from the source element to the target element is a prediction target, selecting, for a first subgraph including the source element, a second subgraph that may be an element of the path, from among one or more second subgraphs.
(B) Based on treatment process pattern structured data and treatment process pattern attribute value data related to a purpose element (e.g., O1) and a result element (e.g., On) compatible with the purpose element and the result element of the process, generating process feature amount attribute value data (e.g., process feature amount [attribute value] 706) that is data including an attribute value related to an element represented by process feature amount structured data (e.g., process feature amount [structure] 605) related to a process feature amount of a target process that is a process including the first subgraph (e.g., G1) and the selected second subgraph (e.g., G2).
(C) Acquiring a result (e.g., an edge prediction table 708) of predicting whether an edge exists between a node in the first subgraph and a node in the second subgraph by inputting, to a causal model for predicting whether an edge exists between the node (e.g., vi) in the first subgraph and the node (e.g., vj) in the second subgraph, a graph feature amount (e.g., a graph feature amount Zg) of structured data as graph data including data of the first subgraph and a value (e.g., a process feature amount in the causal model [E=f(ZG, process feature amount)]) obtained from the process feature amount structured data and the process feature amount attribute value data.
(D) Determining whether or not prediction of the prediction target is completed in an updated process that is the process to which the result is applied (e.g., S2004 in FIG. 20).
(E) When a determination result in (D) is false, performing (A) for the updated process.
(F) When a determination result in (D) is true, outputting structured data representing the updated process.

As a result, the number of combinations is reduced, and external factors of the elements are also taken into consideration in the prediction, thereby making it possible to reduce a computational load of processing for predicting a process represented by generated structured data and improve process prediction accuracy.

The process feature amount may include at least one of a flow feature amount, a path feature amount, and a chain feature amount. The flow feature amount may be a feature amount related to a change caused when the treatment is performed on the purpose element and the result element is obtained. The path feature amount may be a feature amount of the path including the source element and the target element. The chain feature amount may be a feature amount of a position in the path where a pair of the node in the first subgraph and the node in the second subgraph exists. Such a process feature amount contributes to reducing a computational load of processing for predicting a process and improving process prediction accuracy.

For example, the process having the prediction target may be an experimental process. The purpose element and the source element may be the same element and a first substance. The result element and the target element may be the same element and a second substance. The process feature amount may include the flow feature amount. The flow feature amount may be a value (e.g., a Δ value) as a difference from a physical property value of the first substance to a physical property value of the second substance. In addition, the process feature amount may include the path feature amount instead of or in addition to the flow feature amount. The path feature amount may include a value indicating whether an ancestor of the node in the first subgraph is the source element and a value indicating whether a descendant of the node in the second subgraph is the target element. Further, the process feature amount may include the chain feature amount instead of or in addition to at least one of the flow feature amount and the path feature amount. The chain feature amount may be a feature amount of positions of the node in the first subgraph and the node in the second subgraph.

Furthermore, for example, the process having the prediction target may be a measurement process. The purpose element and the source element may be different elements. The result element and the target element may be different elements. The source element may be equipment. The target element may be a detail of the equipment (e.g., specifications such as equipment type). The process feature amount may include the flow feature amount. The flow feature amount may be a value for each of one or more conditions related to a relationship between the purpose element and the result element.

The causal model may be a model generated in training processing and set as a trained decoder (e.g., the decoder 612) . In inference processing, it is not required to generate a causal model, and the causal model set as a decoder may be used. The graph feature amount may be data output from a trained encoder (e.g., the encoder 611) to which structured data as graph data including the data of the first subgraph is input. Such training processing contributes to reducing a computational load of processing for predicting a process and improving process prediction accuracy.

For example, the trained encoder and the trained decoder may be trained using train data and correct answer data in the training processing. The train data may be data including process data as graph data. The causal model may be a model based on process feature amount structured data and process feature amount attribute value data generated for a process represented by the process data based on treatment process pattern structured data and treatment process pattern attribute value data of a treatment process pattern of a treatment applied to the process. The correct answer data may be data including a node pair and presence of an edge in the path from the source element to the target element in the process.

In the training processing, the arithmetic device may generate, for each of the two or more processes, treatment process pattern structured data and treatment process pattern attribute value data of a treatment process pattern, which is a process portion common to the two or more processes, based on process data as structured data for each of the two or more processes. The arithmetic device may generate, for each of the two or more processes, process feature amount structured data and process feature amount attribute value data of the process based on process data including a purpose element, a result element, a source element (e.g., t1), and a target element (e.g., tn), a graph feature amount of the process data, and treatment process pattern structured data and treatment process pattern attribute value data generated for the process. The arithmetic device may generate a causal model for predicting whether an edge exists between nodes of an element based on the process feature amount structured data and the process feature amount attribute value data, and set the causal model as the decoder. The arithmetic device may train the encoder and the decoder using the train data and the correct answer data for each of the two or more processes.

In (A), the first subgraph may be a subgraph including, as one node, a raw material specified from raw material-product data that is data representing a pair of a raw material and a product. The second subgraph may be a subgraph including, as one node, a substance or an operation that may be necessary to obtain a product specified from the raw material-product data. The raw material may be the first substance. The product may be the second substance. Such retrosynthetic analysis makes it possible to predict an experimental process for generating a product from a raw material.

The structuring processing apparatus 100 may further include an interface device (e.g., the communication device 206) communicably connected to a provision destination device. The arithmetic device may provide data including structured data representing the updated process as prediction result data that is data related to a predicted process, to the provision destination device through the interface device. For example, the provision destination device may be the user terminal 101 or an experimental device. For example, in a case where the provision destination device is an experimental device, it is expected to realize a practical application in which the experimental device performs an experiment based on the prediction result data.

Note that, in the above description, the "interface device" may be one or more interface devices. The one or more interface devices may be at least one of the following interface devices.
- One or more input/output (I/O) interface devices. The input/output (I/O) interface device is an interface device for at least one of an I/O device and a remote display computer. The I/O interface device for the display computer may be a communication interface device. At least one I/O device may be any user interface device, for example, an input device such as a keyboard and a pointing device, and an output device such as a display device.
- One or more communication interface devices. The one or more communication interface devices may be one or more communication interface devices of the same type (e.g., one or more network interface cards (NICs)) or two or more communication interface devices of different types (e.g., an NIC and a host bus adapter (HBA)).

In addition, the "memory" is one or more memory devices, which are examples of one or more storage devices, and may typically be a main storage device. At least one memory device in the memory may be a volatile memory device or a nonvolatile memory device.

In addition, the "persistent storage device" may be one or more persistent storage devices, which are examples of one or more storage devices. Typically, the persistent storage device may be a nonvolatile storage device (e.g., an auxiliary storage device), and specifically, for example, may be a hard disk drive (HDD), a solid state drive (SSD), a non-volatile memory express (NVME) drive, or a storage class memory (SCM).

In addition, the "storage device" may be at least the memory among the memory and the persistent storage device.

The "arithmetic device" may be one or more processor devices. At least one processor device may typically be a microprocessor device such as a central processing unit (CPU), but may be another type of processor device such as a graphics processing unit (GPU). At least one processor device may be a single-core or a multi-core. At least one processor device may be a processor core. At least one processor device may be a processor device in a broad sense such as a circuit that is an aggregate of gate arrays described using a hardware description language to perform part or all of processing (e.g., a field-programmable gate array (FPGA), a complex programmable logic device (CPLD), or an application specific integrated circuit (ASIC)).

A plurality of elements represented by treatment process pattern structured data may include elements as purposes and elements as conditions. Specifically, in the example of the radical reaction according to the first embodiment, the raw material (CH₄) and the product (CH₃-CH₃) may correspond to a "purpose" (that is, the purpose is to generate a product from a raw material), and operations such as mixing and irradiation may correspond to a "condition" (that is, the condition for causing the radical reaction is that the operations are performed in the order of mixing -> irradiation). In addition, in the example of the measurement know-how according to the second embodiment, the PET, the DSC measurement, the container, and the type may correspond to a "purpose", and the evaporation, the temperature, and the pressure connected to the condition node may correspond to a "condition".

### Reference Signs List

- 100: structuring processing apparatus

## Claims

1. A structuring processing apparatus comprising:
a storage device that stores treatment process pattern structured data representing a graph of a treatment process pattern for each of one or more treatments and treatment process pattern attribute value data that is data including an attribute value related to an element represented by the treatment process pattern structured data; and
an arithmetic device connected to the storage device,
wherein for each of the one or more treatments, the treatment process pattern is a process portion having a structure common to two or more processes,
for each of the one or more treatments, the treatment process pattern attribute value data includes attribute values related to a purpose element that is an element as a purpose for which the treatment is applied, a result element that is an element as a result of applying the treatment, a source element that is an element to which the treatment is applied, and a target element that is an element when the treatment is applied to the source element, and
the arithmetic device is configured to:
(A) in a process including a purpose element, a result element, and a source element and of which a path from the source element to the target element is a prediction target, select, for a first subgraph including the source element, a second subgraph that may be an element of the path, from among one or more second subgraphs;
(B) based on treatment process pattern structured data and treatment process pattern attribute value data related to a purpose element and a result element compatible with the purpose element and the result element of the process, generate process feature amount attribute value data that is data including an attribute value related to an element represented by process feature amount structured data related to a process feature amount of a target process that is a process including the first subgraph and the selected second subgraph;
(C) acquire a result of predicting whether an edge exists between a node in the first subgraph and a node in the second subgraph by inputting, to a causal model for predicting whether an edge exists between the node in the first subgraph and the node in the second subgraph, a graph feature amount of structured data as graph data including data of the first subgraph and a value obtained from the process feature amount structured data and the process feature amount attribute value data;
(D) determine whether or not prediction of the prediction target is completed in an updated process that is the process to which the result is applied;
(E) when a determination result in (D) is false, perform (A) for the updated process; and
(F) when a determination result in (D) is true, output structured data representing the updated process.

2. The structuring processing apparatus according to claim 1, wherein
the process feature amount includes at least one of a flow feature amount, a path feature amount, and a chain feature amount,
the flow feature amount is a feature amount related to a change caused when the treatment is performed on the purpose element and the result element is obtained,
the path feature amount is a feature amount of the path including the source element and the target element, and
the chain feature amount is a feature amount of a position in the path where a pair of the node in the first subgraph and the node in the second subgraph exists.

3. The structuring processing apparatus according to claim 1, wherein
the process having the prediction target is an experimental process,
the purpose element and the source element are the same element and a first substance, and
the result element and the target element are the same element and a second substance.

4. The structuring processing apparatus according to claim 3, wherein
the process feature amount includes the flow feature amount, and
the flow feature amount is a value as a difference from a physical property value of the first substance to a physical property value of the second substance.

5. The structuring processing apparatus according to claim 1, wherein
the process feature amount includes the path feature amount, and
the path feature amount includes a value indicating whether an ancestor of the node in the first subgraph is the source element, and a value indicating whether a descendant of the node in the second subgraph is the target element.

6. The structuring processing apparatus according to claim 1, wherein
the process feature amount includes the chain feature amount, and
the chain feature amount is a feature amount of positions of the node in the first subgraph and the node in the second subgraph.

7. The structuring processing apparatus according to claim 1, wherein
the process having the prediction target is a measurement process,
the purpose element and the source element are different elements,
the result element and the target element are different elements,
the source element is equipment, and
the target element is a detail of the equipment.

8. The structuring processing apparatus according to claim 7, wherein
the process feature amount includes the flow feature amount, and
the flow feature amount is a value for each of one or more conditions related to a relationship between the purpose element and the result element.

9. The structuring processing apparatus according to claim 1, wherein
the causal model is a model generated in training processing and set as a trained decoder, and
the graph feature amount is data output from a trained encoder to which structured data as graph data including the data of the first subgraph is input.

10. The structuring processing apparatus according to claim 9, wherein
the trained encoder and the trained decoder are trained using train data and correct answer data in the training processing,
the train data is data including process data as graph data,
the causal model is a model based on process feature amount structured data and process feature amount attribute value data generated for a process represented by the process data based on treatment process pattern structured data and treatment process pattern attribute value data of a treatment process pattern of a treatment applied to the process, and
the correct answer data is data including a node pair and presence of an edge in the path from the source element to the target element in the process.

11. The structuring processing apparatus according to claim 10, wherein
the arithmetic device is configured to, in the training processing:
generate, for each of the two or more processes, treatment process pattern structured data and treatment process pattern attribute value data of a treatment process pattern, which is a process portion common to the two or more processes, based on process data as structured data for each of the two or more processes;
generate, for each of the two or more processes, process feature amount structured data and process feature amount attribute value data of the process based on process data including a purpose element, a result element, a source element, and a target element, a graph feature amount of the process data, and treatment process pattern structured data and treatment process pattern attribute value data generated for the process;
generate a causal model for predicting whether an edge exists between nodes of an element based on the process feature amount structured data and the process feature amount attribute value data;
set the causal model as the decoder; and
train the encoder and the decoder using the train data and the correct answer data for each of the two or more processes.

12. The structuring processing apparatus according to claim 3, wherein
in (A),
the first subgraph is a subgraph including, as one node, a raw material specified from raw material-product data that is data representing a pair of a raw material and a product,
the second subgraph is a subgraph including, as one node, a substance or an operation that may be necessary to obtain a product specified from the raw material-product data,
the raw material is the first substance, and
the product is the second substance.

13. The structuring processing apparatus according to claim 1, further comprising an interface device communicably connected to a provision destination device,
wherein the arithmetic device provides data including structured data representing the updated process as prediction result data that is data related to a predicted process to the provision destination device through the interface device.

14. The structuring processing apparatus according to claim 1, wherein the plurality of elements represented by the treatment process pattern structured data includes an element as a purpose and an element as a condition.

15. A structuring processing method performed by a computer, the method comprising:
(A) in a process including a purpose element, a result element, and a source element and of which a path from the source element to the target element is a prediction target, selecting, for a first subgraph including the source element, a second subgraph that may be an element of the path, from among one or more second subgraphs;
(B) based on treatment process pattern structured data and treatment process pattern attribute value data related to a purpose element and a result element compatible with the purpose element and the result element of the process, generating process feature amount attribute value data that is data including an attribute value related to an element represented by process feature amount structured data related to a process feature amount of a target process that is a process including the first subgraph and the selected second subgraph;
(C) acquiring a result of predicting whether an edge exists between a node in the first subgraph and a node in the second subgraph by inputting, to a causal model for predicting whether an edge exists between the node in the first subgraph and the node in the second subgraph, a graph feature amount of structured data as graph data including data of the first subgraph and a value obtained from the process feature amount structured data and the process feature amount attribute value data;
(D) determining whether or not prediction of the prediction target is completed in an updated process that is the process to which the result is applied;
(E) when a determination result in (D) is false, performing (A) for the updated process; and
(F) when a determination result in (D) is true, outputting structured data representing the updated process,
wherein there are treatment process pattern structured data representing a graph of a treatment process pattern for each of one or more treatments and treatment process pattern attribute value data that is data including an attribute value related to an element represented by the treatment process pattern structured data,
for each of the one or more treatments, the treatment process pattern is a process portion having a structure common to two or more processes, and
for each of the one or more treatments, the treatment process pattern attribute value data includes attribute values related to a purpose element that is an element as a purpose for which the treatment is applied, a result element that is an element as a result of applying the treatment, a source element that is an element to which the treatment is applied, and a target element that is an element when the treatment is applied to the source element.
